(19) Europäisches Patentamt
European Patent Office
Office européen des brevets

(11) Publication number: 0 071 991
B1

# (12) EUROPEAN PATENT SPECIFICATION

(45) Date of publication of patent specification: 14.05.86

(51) Int. Cl.$^4$: G 01 N 33/533

(21) Application number: 82107102.4

(22) Date of filing: 06.08.82

## (54) Improved fluoro immuno assay system.

(30) Priority: 10.08.81 US 291793

(43) Date of publication of application:
16.02.83 Bulletin 83/07

(45) Publication of the grant of the patent:
14.05.86 Bulletin 86/20

(84) Designated Contracting States:
AT DE FR GB IT

(56) References cited:
GB-A-2 014 300
GB-A-2 063 469
US-A-4 144 452
US-A-4 235 869

JOURNAL OF PHYSICS E.; SCIENTIFIC INSTRUMENTS, vol. 7, no. 4, April 1974, E. KARJALAINEN et al.: "A multi-channel photometer for chemical analysis", pages 241-243

CLINICAL CHEMISTRY, vol. 25, no. 4, April 1979, EASTON, PENNSYLVANIA (US), C.H. McMURRAY et al.: "Multi-channel probe colorimeter for use with the Micro-ELISA test, which makes use of disposable flat-bottom microhemagglutination plates"

(73) Proprietor: BIO-DIAGNOSTICS, INC.
2100 Road to Six Flags
Arlington Texas 76011 (US)

(72) Inventor: Hendrix, John L.
4962 Lakeland Drive Marietta
Georgia 30067 (US)

(74) Representative: Patentanwälte Dr. Solf & Zapf
Schlossbleiche 20 Postfach 13 01 13
D-5600 Wuppertal 1 (DE)

(56) References cited:
BIOCHEMISTRY, vol. 20, no. 26, December 1981, K.A. WRIGHT et al.: "Solution properties of synthetic chlorophyllide- and bacteriochlorophyllide-A.pomyoglobin complexes", pages 7546-7756

CHEMICAL ABSTRACTS, vol. 81, no. 5, August 5, 1974, page 312, abstract no. 24029q, COLUMBUS OHIO (US), J.P. KRAEHENBUHL et al.: "Preparation and characterization of an immunoelectron microscope tracer consisting of a heme-octapeptide coupled to Fab."

EP 0 071 991 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical Field

The present invention relates to immuno assay systems and in particular to a method of conducting fluoro immuno assays in which the fluorescent markers provide a relatively large Stokes shift from the excitation wavelength to the wavelength of fluorescent emission.

Background of the Invention

Immuno assays are used in a wide variety of applications in the field of medicine. Such assays are tests to determine the concentrations of various substances present in blood samples taken from a patient. There are many known assays for particular substances. One of the more common classes of assays is to determine the concentration of particular hormones in a patient's blood. For example, thyroxine, also called T4, is a hormone which regulates human metabolism. It is desirable to screen all newborn babies for a shortage of T4 since such a condition can cause irreversible mental retardation if not treated. Similarly, assays for triidothyroxine also known as T3 may be used to detect hyperthyroid conditions in patients.

Assays also exist for the presence of viruses and bacteria in a patient. Also, some modern drugs which are used to treat patients become toxic if allowed to accumulate in high concentrations in the patient's blood and, ultimately, do more harm than good. One example is the drug digoxin which is used to regulate the heart beat in cardiac patients. The problem of treatment with digoxin is that there is a wide variation from patient to patient in the amount of the drug required to produce particular concentrations in the bloodstream and excess concentrations of digoxin are toxic.

Modern immuno assay systems fall generally into three categories: (1) radio immuno assays; (2) enzyme immunoassays; and (3) fluoro immuno assays. The common feature of each is the use of marked or labelled standard solutions. A typical immuno assay scheme involves the preparation of an antibody specific to the substance (antigen) for which the test is to be conducted. Samples of the antigen are prepared by the tester which have the marker in question attached to each molecule. In the case of radio immuno assays (RIA) the labelling agent is a radioactive substance. In the case of fluoro immuno assays (FIA), the labelling agent is a material having known fluorescent qualities. Because enzyme immunoassays are measured spectrophotometrically, they are not as sensitive as radioimmunoassays or fluoro immuno assays.

The next step is to provide a dose response curve which is arrived at by mixing the labelled or antigen solution with differing concentrations of a standard unmarked antigen solution and allowing these various mixtures to compete for antibody sites in a container such as a microtiter well which has been coated with a specific antibody. The marked and unmarked antigens in the mixtures compete for the antibody sites and become attached to the antibody sites in proportion to their presence in the mixture.

The remainder of the mixture is removed from the well containing the antibodies by aspiration or some other method and then tested for the presence of the labelled antigens. Since the labelled solution was mixed with various known concentrations of unlabelled antigen, a dose response curve may be drawn, either by hand or with the aid of a computer, to correlate the output of the label detector to the concentration of the unlabelled antigens for the particular sample of marked antigen solution being used. Thus, the dose response curve provides a way of directly translating the amount of marked antigen attached to the antibody sites to the concentration of the unmarked antigen with which the marked solution was mixed.

When this has been accomplished, samples of the marked solution will be mixed with samples of patient's serum, and the marked antigen molecules will compete with the patient antigen molecules for the antibody sites. In the same fashion recited above, the patient antigen molecules and the labelled antigen molecules will compete for the available antibody sites and will be successful in proportion to their relative concentrations in the solution.

When the remainder of the solution is removed, the container is tested for the presence of the label. By using the dose response curve (which is for the particular marked solution being used) the amount of the labelled antigen which remains attached to the antibody sites will provide a direct indication off the dose response curve of the concentration of the particular antigen in the patient serum sample.

In the case of RIA, the most common label used is a radioactive isotope of iodine, $^{125}I$. In testing for the amount of labelled antigen attached to the antibody sites, the radioactive emissions from the samples must be counted in order to get a reading off the dose response curve indicative of the concentration of the antigen in the patient serum.

Radio immuno assays have become very popular in that they provide assays of very good reliability and sensitivity. The fundamental drawback of radio immuno assays is that they are quite expensive. First, the apparatus required to test for the presence of the radioactivity is complex and expensive. Secondly, the particular isotope of iodine used has a radioactive half life of sixty days. Solutions of antigens labelled with radioactive iodine must be used very shortly after preparation since their shelf life is severely limited by the rapid radioactive decay of the marker substance. It will thus be appreciated that the economics of distribution are such that only small amounts are provided to each user at any one time and must be shipped very rapidly from point of preparation to point of end use.

Also, there has been a growing reluctance on the part of many shippers, particularly airlines to transport radioactive materials.

In order to overcome these basic drawbacks of RIA techniques, fluoro immuno assays have been created. The main advantage of fluoro immuno assays over radio immuno assays is that the fluorescent compounds used as labels to be attached to antigens are very stable relative to the short radioactive half life of markers used in RIAs. Secondly, the expense of the testing apparatus for FIAs is usually less than that for RIAs and the external problems of handling radioactive materials do not arise.

The basic principle of using fluorescent labels in fluoro immuno assays is that certain materials, when illuminated by radiation in the spectrum around visible light, will emit radiation of a lower frequency (longer wavelength) in response to being so illuminated. For most fluorescent materials used in FIAs, the emitted radiation is in the spectrum of visible light, and thus detectors for detecting light may be used to ascertain the presence of the fluorescent label.

It is known that for such fluorescent materials, the frequency of the fluorescent emission is lower than the frequency of the radiation which causes the material to fluoresce. It therefore follows that the wavelength of the fluorescent emission is longer than the wavelength of the radiation illuminating the material. The difference between the wavelength of fluorescent emission and the wavelength of illumination (excitation) is referred to as the Stokes' shift. Each of the materials used as a fluorescent label has particular characteristics of required wavelength of excitation and resulting wavelength of fluorescent emission, and thus has a characteristic Stokes' shift.

There are two major drawbacks to prior art fluoro immuno assays: (1) the fluorescent marker materials used have been characterized by a relatively low Stokes' shift on the order of twenty to thirty-five nanometers; and (2) the wavelengths of fluorescent emission for the fluorescent markers are very close to the wavelengths of auto fluorescence exhibited by components which are often present in the patient's serum.

The first drawback mentioned above is one which requires very sensitive detectors and complex optical apparatus to distinguish between light having a wavelength characteristic of the fluorescent material and the light used to excite the fluorescent material. Because of the low Stokes shift, it is difficult and expensive to design light sensors which will respond to the wavelength of fluorescence and be relatively insensitive to the wavelength of the excitation light. In order to overcome this problem, many prior art fluoro immuno assay devices have used expensive diffraction gratings inserted between the sample containing the fluorescent labels and the optical sensor. These are placed so that they are orthogonal to the direction of a beam of light at the excitation wavelength.

The second drawback noted above reduces the sensitivity and reliability of prior art fluoro immuno assays relative to RIAs. The presence of autofluorescing components in the serum require extra precautions and extra steps to assure that these autofluorescing substances are removed from the sample containing the antibodies before the ultimate test for presence of fluorescent material is made. This increases the complexity and expense of preparing the samples. Furthermore, it is difficult to assure removal of all of the auto fluorescing substances and thus the reliability of previous fluoro immuno assays has tended to be less than that of radio immuno assays.

## Summary of the Invention

The present invention overcomes the drawbacks of previous fluoro immuno assays by providing fluorescent labelling material characterized by a relatively high Stokes shift. Furthermore, the assays of the present invention provide fluorescent labelling materials which fluoresce at wavelengths considerably longer than the characteristic wavelengths of most autofluorescing substances to be found in patient serum samples.

Thus, the present invention, by using fluorescent labelling materials characterized by both a high Stokes shift and a fluorescent wavelength far removed from the characteristic wavelengths of autofluorescing proteins, can be used in a FIA apparatus in which the excitation light source may be placed directly above the sample, such as a well in a microtiter plate; with the light sensors being placed directly below the well.

Since the optical transmission path requires in connection with the invention no diffraction gratings, expensive lenses etc., the present invention makes possible to use a simple arrangement for duplicating the optical path several times over. This provides another important advantage of the present invention, the ability to conduct large numbers of tests with a single apparatus at one time, and to automate the process of computing the dose response curve and providing quantitative results of tests on patient samples.

Accordingly, it is an object of the present invention to provide an improved fluoro immuno assay system, so that a plurality of tests may be conducted automatically, at one time, under the control of a computer.

It is a further advantage of the present invention, that an automated arrangement for conducting fluoro immuno assay tests for which a plurality of samples may be tested, in sequence under the control of a computer can be used, whereby the results are multiplexed to the computer and the dose response curve, as well as the quantified results of a plurality of patient samples, are provided very rapidly.

It is a further object of the present invention to provide fluoro immuno assays for which the optical

sensing signal path is characterized by at least a ten decibel drop in sensitivity from the characteristic wavelength of the label's fluorescence and the characteristic wavelength of fluorescence from common serum components.

It is a further advantage of the present invention, that fluorescent labelling agents can be provided useful in FIA procedures which labelling agents have relatively large Stokes and wavelengths of fluorescent emission relatively greatly removed from the wavelength of autofluorescence exhibited by protein molecules often associated with FIA procedures.

These and other objects, features and advantages of the present invention will become apparent after a review of the following detailed description of the disclosed embodiment and the appended drawings and claims.

## Brief Description of the Drawing

Fig. 1 is a pictorial view of an apparatus which can be used in connection with the present invention.

Fig. 2 is a block diagram of the apparatus according Fig. 1,

Fig. 3 is a block diagram of a type of apparatus, which can be used in connection with the present invention.

Fig. 4 is a block diagram of a multiplexing arrangement of the apparatus according Fig. 1.

Fig. 5 is a frequency response graph of a photodetector used in constructing the apparatus according Fig. 1.

## Detailed Description

In using the present invention, an assay reagent is labelled with a fluorescent labelling agent having a Stokes shift of not less than 150 nanometers to provide a labelled assay reagent. A labelled assay reagent is obtained by conjugating the reagent with a labelling agent. The present invention is applicable to virtually all assay reagents which are capable of conjugation with the fluorescent labelling agent of the present invention. Such assay reagents include, for example, antigens, antibodies, hormones, virus particles, haptens, bacterial components, drugs, monoclonal antibodies, anti-antibodies (also known as double antibodies or second antibodies), immuno globulins and proteins. Specific assay reagents which are useful in the present invention include thyroxine, triiodothyronine, thyroid stimulating hormone, thyroxine binding globulin, thyrotropin releasing hormone, digoxin, gentamicin, tobramycin, phenytoin, theophyllin, tetracycline, Hepatitis B surface antigen, Hepatitis B core antigen, Hepatitis A antigen, carcinoembryonic antigen, prostatic acid phosphatase and human chorionic gonadotropin.

The fluorescent labelling agents which are useful in the present invention include chlorophylls and porphyrins which have a Stokes shift of not less than approximately 150 nanometers.

Chlorophylls are the green pigments that are extractable by organic solvents from all autotropic and chemoautotrophic plants. Approximately ten chlorophylls have been isolated from the green parts of various plants. The most abundant green component is chlorophyll a followed by chlorophyll b, chlorophyll $c_1$ and $c_2$, chlorophyll d, protochlorophyll, bacteriochlorophylls and chlorobium chlorophylls. All of the foregoing chlorophylls have Stokes shifts greater than 150 nanometers and are useful in the present invention.

Chlorophyll a is a magnesium-complexed dihydroporphyrin with two additional hydrogen atoms at positions C—7 and C—8. Chlorophyll a is the only green pigment formed in yellow-green, blue-green and some red algae and is found in combination with small quantities of other chlorophylls in higher plants, such as *Euglena,* diatoms, dinoflagellates, green algae, brown algae and a few red algae. Chlorophyll a has the following structural formula:

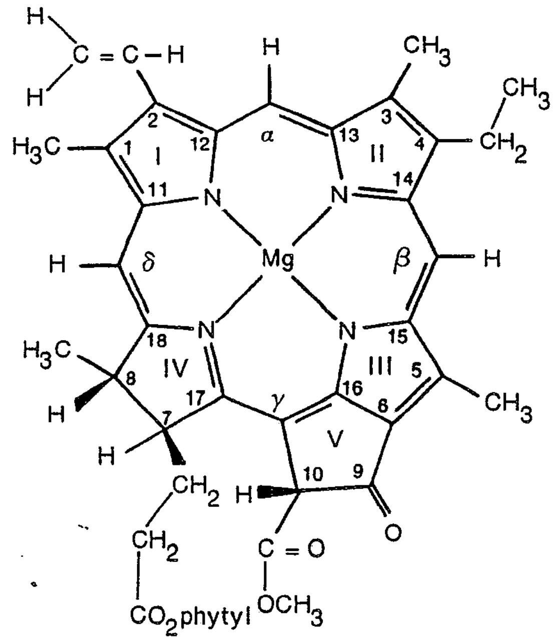

Chlorophyll a

phytyl = 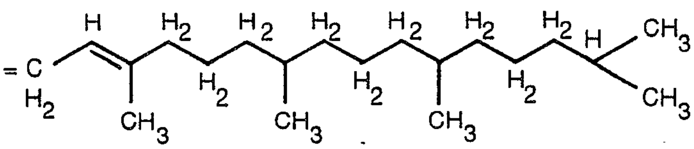

Chlorophyll b is also a dihydroporphyrin, but it differs from chlorophyll a by replacement of the methyl group at C—3 by a formyl group. Chlorophyll b occurs as the major green pigment in vascular plants, green algae and *Euglena.* Chlorophyll b has the following structural formula:

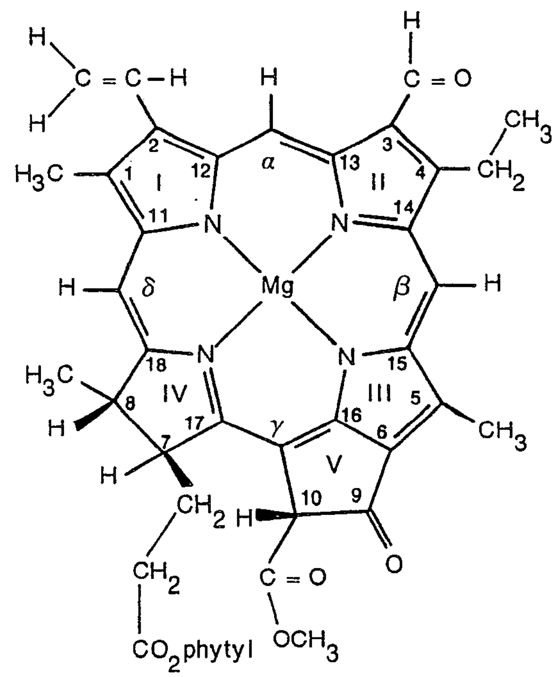

Chlorophyll b

Chlorophyll c is found in diatoms, dinoflagellates, brown algae and in certain symbiotic algae of sea anemones. Chlorophyll c occurs as a mixture of two compounds, Chlorophyll $c_1$ and $c_2$. Chlorophyll $c_1$ (magnesium tetradehydropheoporphyrin $a_5$ monomethyl ester) has the following structure formula:

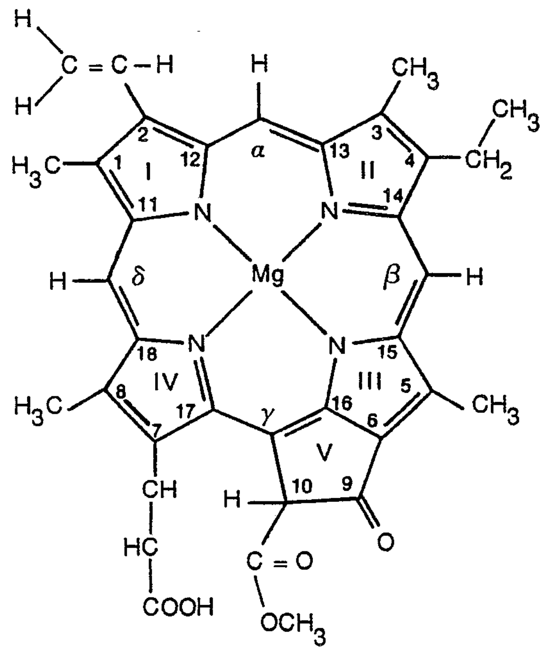

Chlorophyll $c_1$

Chlorophyll $c_2$ (magnesium hexadehydropheoporphyrin $a_5$ monomethyl ester) has the following structural formula:

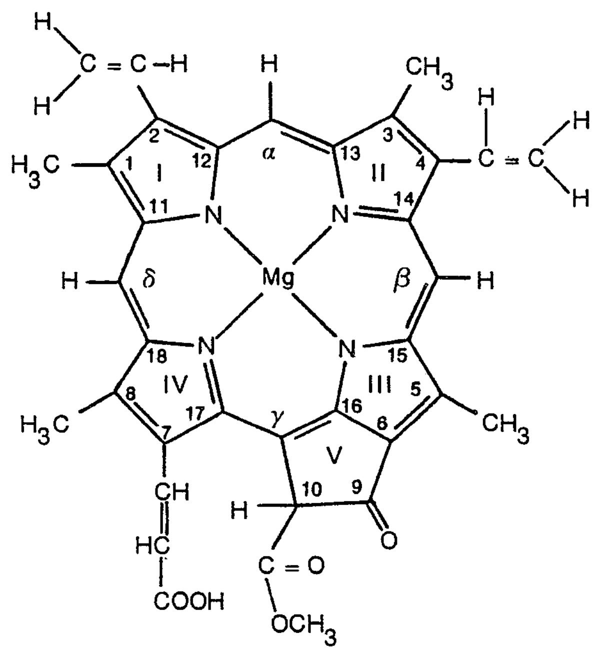

Chlorophyll $c_2$

Protochlorophyll may occur in either of two forms, the normal phytol ester or the free acid, protochlorophyllide. Protochlorophyll can be prepared from the oxidation of chlorophyll a with 2,3-dichloro-5,6-dicyano-1,4-benzoquinone and occurs naturally in minute quantities in yellow, etiolated seedlings grown in the dark and in the inner seed coats of cucumbers, squash and pumpkin seeds. Protochlorophyll has the following structural formula:

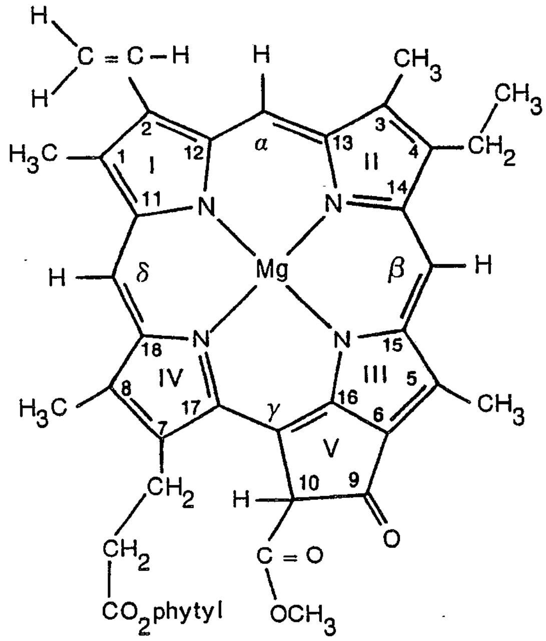

Protochlorophyll

Chlorophyll d is a dihydroporphyrin (2-desvinyl-2-formyl-chlorophyll a) and occurs as the minor chlorophyll accompanying chlorophyll a in some red algae. Chlorophyll d has the following structural formula:

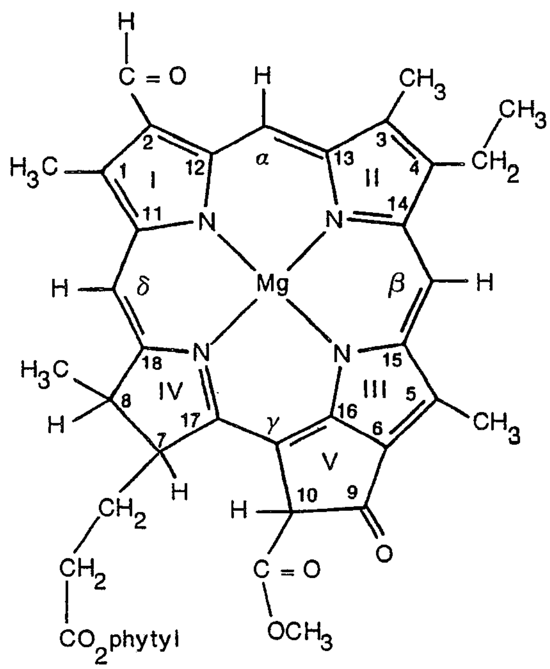

Chlorophyll d

The precise composition of chlorobium chlorophylls is unknown. Chlorobium chlorophyll is thought to be a mixture of six different chlorophylls differing by side chain modifications. Two different chlorobium chlorophylls have been identified and are named according to the wavelength of the red absorption maxima in ether: chlorobium chlorophyll 660 and chlorobium chlorophyll 650. Chlorobium chlorophylls, originally named bacterioviridin are found in the green chemoautotrophic sulfur bacteria where they are accompanied by a small amount of bacteriochlorophyll a.

Bacteriochlorophylls are the principal chlorophylls of chemoautotropic purple sulfur bacteria (Athiorhodaceae, Thiorhodaceae and Hyphomicrobiaceae). Three chlorophyll entities are known to occur in these organisms: bacteriochlorophyll $a_p$, bacteriochlorophyll $a_{gg}$ and bacteriochlorophyll b.

Bacteriochlorophyll a has the following structural formula:

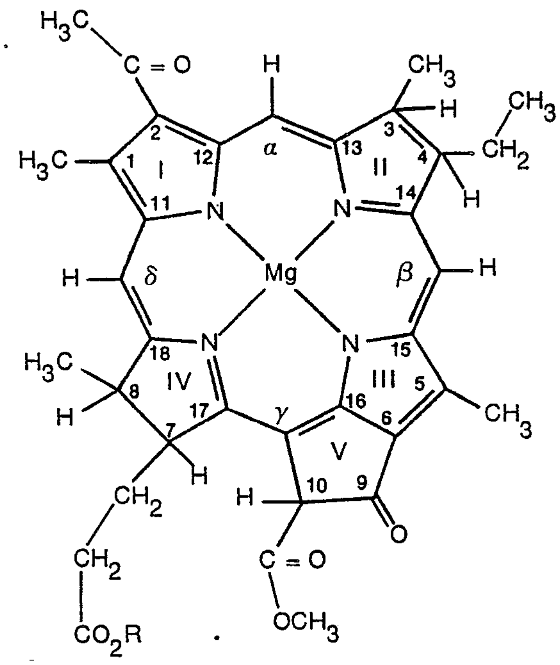

wherein R is:

Bacteriochlorophyll $a_p$
R = phytyl =

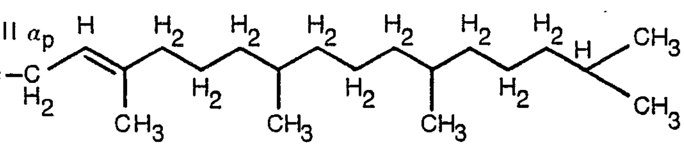

Bacteriochlorophyll $a_{gg}$
R=geronylgeronyl =

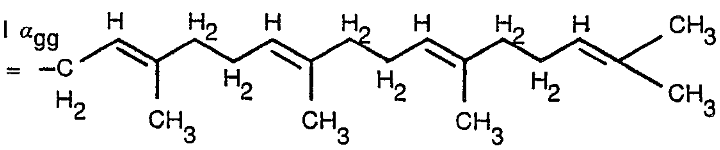

Bacteriochlorophyll $a_p$ and bacteriochlorophyll $a_{gg}$ can be obtained from *Rhodopseudomonas palustris* (ATCC 17001) and *Rhodospirillum photometricum* (NTHC 13). Bacteriochlorophyll $a_{gg}$ is isolated as the principal green pigment from *Rhodospirillum rubrum.* Bacteriochlorophyll b is the major chlorophyll found in the photosynthetic bacterium *Rhodopseudomonas viridis.*

Bacteriochlorophyll b has the following structural formula:

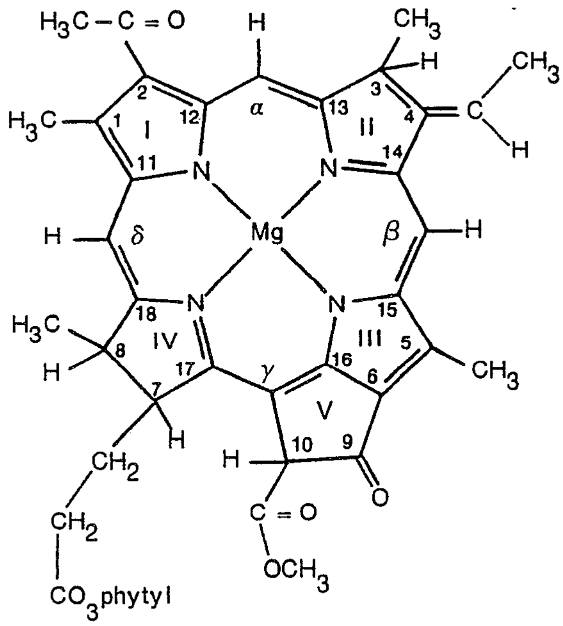

Bacteriochlorophyll b

Although all of the foregoing chlorophylls have Stokes shifts in excess of 150 nanometers, bacteriochlorophyll b is preferred due to its relative ease of preparation and a Stokes shift of approximately 250 nanometers. Other chlorophylls are also useful in the present invention as long as they have Stokes shifts of not less than approximately 150 nanometers.

The absorption maxima and specific coefficients of various chlorophylls are shown in Table 1 below:

TABLE 1

Absorption Maxima ($\lambda_{max}$) and Specific Absorption Coefficients ($c$) of the Chlorophylls

| Compound | Solvent | $\lambda_{max}$ red (nm) | $c \times 10^{-3}$ red | $\lambda_{max,}$ blue (nm) | $c \times 10^{-3}$ blue | Ratio abs. blue/red |
|---|---|---|---|---|---|---|
| Chl *a* | $Et_2O$ | 660.5 | 96.6 | 428.5 | 125.1 | 1.30 |
| Chl *a* | Acetone | 663.0 | 92.6 | 430.0 | 106.0 | — |
| Chl *a* | Acetone (80%) | 665.0 | 90.8 | 433.0 | 101.5 | 1.21 |
| Chl *a* | Et.OH (96%) | 665.0 | 83.4 | 432.0 | 83.2 | 1.00 |
| Chl *b* | $Et_2O$ | 642.0 | 61.8 | 442.5 | 175.3 | 2.84 |
| Chl *b* | Acetone | 645.0 | 51.8 | 455.0 | 146.9 | — |
| Chl *b* | Acetone (80%) | 648.5 | 52.5 | 460.0 | 148.0 | 3.05 |
| Chl *b* | EtOH (96%) | 649.0 | 44.2 | 464.0 | 118.4 | 2.68 |
| DChl *a* | $Et_2O$ | 659.0 | 88.6 | 428.0 | 116.1 | 1.31 |
| DChl *b* | $Et_2O$ | 640.5 | 57.9 | 451.0 | 165.7 | 2.86 |
| Chls *c* | $Et_2O$ | 626.5 | 29.6 | 447.3 | 262.1 | 9.10 |
| Chls *c* | Acetone | 628.0 | 15.8 | 442.0 | 115.9 | 7.52 |
| Chl $c_1$ | Pyridine | 639.6 | 35.0 | 461.5 | 346.0 | 9.90 |
| Chl $c_1$ | Acetone | 629.1 | 39.2 | 446.1 | 348.0 | 8.89 |
| Chl $c_2$ | Pyridine | 641.5 | 31.8 | 466.0 | 459.0 | 14.45 |
| Chl $c_2$ | Acetone | 629.6 | 37.2 | 444.6 | 321.0 | 8.62 |
| Chl *d* | $Et_2O$ | 686.0 | 117.8 | 445.0 | 97.8 | ~0.88 |
| Protochl | $Et_2O$ | 623.0 | 36.9 | 432.0 | 305.9 | 8.14 |
| Protochl | Acetone | 623.0 | 34.9 | 432.0 | 270.5 | — |
| Bchl *a* | $Et_2O$ | 772.0 | 105.0 | 358.0 | 93.7 | 0.87 |
| Bchl *a* | Acetone | 775.0 | 22.1 | 358.0 | 44.2 | — |
| Bchl *b* | Acetone | 795.0 | — | 368.0 | — | ~0.93 |
| Chlorobium Chls 660 | $Et_2O$ | 660.0 | 95.4 | 431.0 | 143.0 | ~1.58 |
| Chlorobium Chls 650 | $Et_2O$ | 650.0 | 113.5 | 425.0 | 146.0 | ~1.51 |

Alteration products or derivatives of chlorophylls are also useful in the present invention and include: chlorophyll isomers, e.g. chlorophylls a, b and d and bacteriochlorophylls are diastereomeric at C—10; chlorophyllides; chlorophyllide esters; oxidized chlorophylls, allomerized chlorophylls, pyrochlorophylls, 9-deoxo-9-hydroxychlorophylls; reaction products with amines to provide substituted chlorin-6-amides; chlorophylls (saponification of chlorophylls in strongly alkaline solutions in alcohol); 2-devinyl-2-acetyl-chlorophyll a.

The structures and properties of various chlorophyll a derivatives is shown in Table 2.

TABLE 2

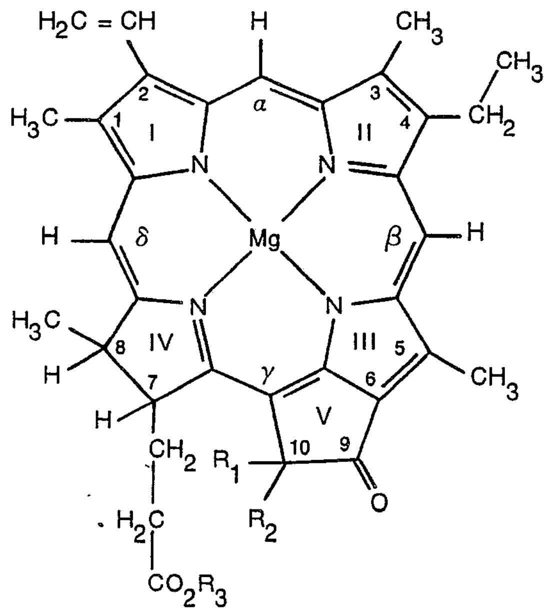

Chlorophyll *a* (by chromatography)

Formula: $C_{55}H_{72}O_5N_4Mg$ MW: 892.5350

Structure: $R_1$ = —H. $R_2$ = —$CO_2CH_3$. $R_3$ = $C_{20}H_{38}$

Chlorophyll *a'* (by heating in pyridine)

Formula: $C_{55}H_{72}O_5N_4Mg$ MW: 892.5350

Structure: Same as Chl *a* with inversion around C—10

Abs. max. ether: 661.0 nm; 428.5 nm; ratio: 1.29

Chlorophyllide *a* (by enzymatic hydrolysis of the phytyl group)

Formula: $C_{35}H_{34}O_5N_4Mg$ MW: 614.2387

Structure: $R_1$ = —H. $R_2$ = —$CO_2CH_3$. $R_3$ = —H

Abs. max. ether: 660.5 nm; c. 131,000; 428 nm; c. 88.200

Methyl chlorophyllide *a* (by enzymatic hydrolysis in methanol)

Formula: $C_{35}H_{36}O_5N_4Mg$ MW: 628.2533

Structure: $R_1$ = —H. $R_2$ = —$CO_2CH_3$. $R_3$ = —$CH_3$

Abs. max. ether; 660.5 nm; c, 83,000; 427.5 nm; ratio: 1.30

Ethyl chlorophyllide *a* (by enzymatic hydrolysis in ethanol)

Formula: $C_{37}H_{26}O_5N_4Mg$ MW: 642.2690

Structure: $R_1$ = —H, $R_2$ = —$CO_2CH_3$. $R_3$ = —$CH_2CH_3$

Abs. max. ether: 660 nm; c, 89.3000; 427.5 nm; c, 119.000; ratio: 1.33

10-Hydroxychlorophyll *a* (by enzymatic oxidation or nonenzymatic allomerization)

Formula: $C_{55}H_{72}O_6N_4Mg$ MW: 908.5301

TABLE 2 (continued)

Structure: $R_1$ = —OH. $R_2$ = —$CO_2CH_3$. $R_3$ = $C_{20}H_{38}$

Abs. max. ether: 660.5 nm; 428 nm; ratio: 1.29

10-Methoxychlorophyll *a* (by allomerization)

Formula: $C_{56}H_{74}O_6N_4Mg$ MW: 922.5457

Structure: $R_1$ = —$OCH_3$. $R_2$ = —$CO_2CH_3$. $R_3$ = $C_{20}H_{38}$

Abs. max. ether: 660.5 nm; 428.5 nm

10-Methoxylactone chlorophyll *a* (by allomerization)

Formula: $C_{56}H_{74}O_7N_4Mg$ MW: 938.5406

Structure: $R_1$ = —$OCH_3$. $R_2$ = —$CO_2CH_3$. $R_3$ = $C_{20}H_{38}$

Ring V = 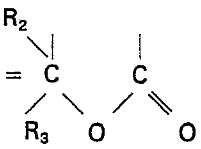

Abs. max. ether: 656 nm; 416 nm; ratio: 1.82

Pyrochlorophyll *a* (by prolonged heating in pyridine)

Formula: $C_{53}H_{70}O_3N_4Mg$ MW: 834.5297

Structure: $R_1$ = —H. $R_2$ = —H. $R_3$ = —$C_{20}H_{38}$

Abs. max. ether: 659.5 nm; c, 80,000, 429.0 nm; ratio: 1.49

Methyl pyrochlorophyllide *a* (by enzymatic hydrolysis and heating in pyridine)

Formula: $C_{34}H_{34}O_3N_4Mg$ MW: 570.2480

Structure: $R_1$ = —H. $R_2$ = —H. $R_3$ = —$CH_3$

Abs. max. ether: 659.0 nm; c, 72,000; 428.0 nm; ratio: 1.52

9-Deoxo-9-hydroxychlorophyll *a* (by reduction with sodium borohydride)

Formula: $C_{55}H_{74}O_5N_4Mg$ MW: 894.5506

Structure: $R_1$ = —H. $R_2$ = —$CO_2CH_3$. $R_3$ = —$C_{20}H_{38}$ —H and —OH in place of =O at C—9

Abs. max. ether: 655.0 nm; c, 53,000; 397 nm; c, 172,000; ratio: 3.25

Chlorophyll *a* amine products (by reaction with amines)

General Formula: $C_{55}H_{72}O_5N_3Mg$ (NR'R'')

Structure: $R_1$ = —H. $R_2$ = —$CO_2CH_3$. $R_3$ = —$C_{20}H_{38}$

Ring V cleaved = 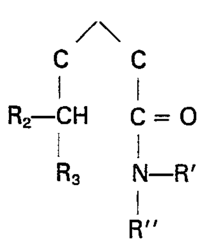

TABLE 2 (continued)

Abs. spectra of Chl *a* + *n*-propylamine, ether: 641 nm; 416 nm

2-Devinyl-2-acetylchlorophyll *a* (by oxidation of bacteriochlorophylls)

Formula: $C_{55}H_{72}O_6N_4Mg$ MW: 908.5301

Structure: $R_1$ = —H. $R_2$ = —$CO_2CH_3$. $R_3$ = —$C_{20}H_{38}$

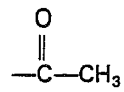 in place of 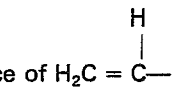 at position 2

Abs. max. acetone: 678.5 nm; 437 nm; ratio: 2.08

---

The structure and properties of various magnesium-free derivatives of chlorophyll a is shown in Table 3.

TABLE 3
Structure and Properties of Magnesium-Free Chlorophyll *a* Derivatives

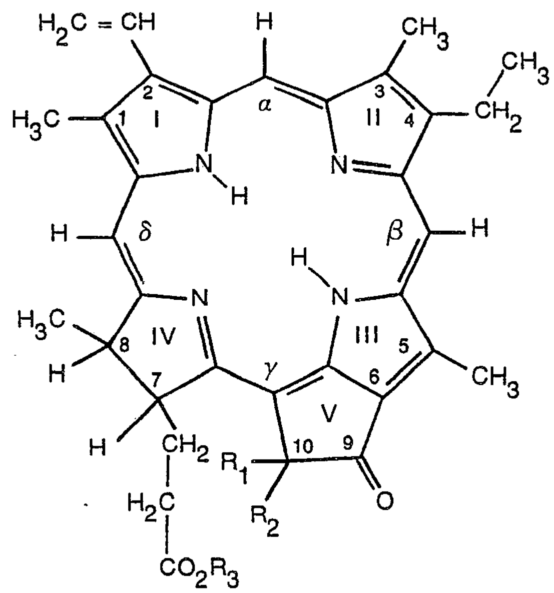

Pheophytin *a* (by treatment with aqueous mineral acid)

Formula: $C_{55}H_{71}O_5N_4$ MW: 870.5657

Structure: $R_1$ = —H. $R_2$ = —$CO_2CH_3$. $R_3$ = —$C_{20}H_{38}$

Abs. max. ether: 667.0 nm; c. 76.900; 409 nm; c, 156.000; ratio: 2.09

Pheophorbide *a* (by refluxing in acidic acetone)

Formula: $C_{35}H_{36}O_5N_4$ MW: 592.2684

Structure: $R_1$ = —H. $R_2$ = —$CO_2CH_3$. $R_3$ = —H

Abs. max. ether: 667.0 nm; C, 70.200; 408.5 nm; c, 141.500; ratio: 2.07

Methyl pheophorbide *a* (by refluxing in acidic methanol)

Formula: $C_{36}H_{30}O_5N_4$ MW: 606.2841

Structure: $R_1$ = —H. $R_2$ = —$CO_2CH_3$. $R_3$ = —$CH_3$

TABLE 3 (continued)

Abs. max. ether: 667.0 nm; c, 59.200; 408.5 nm; c, 135.000; ratio: 2.07

Pyropheophytin *a* (by prolong heating in pyridine and acidification)

Formula: $C_{53}H_{72}O_3N_4$ MW: 812.5603

Structure: $R_1$ = —H. $R_2$ = —H. $R_3$ = —$C_{20}H_{38}$

Abs. max. ether: 667.0 nm; c, 59.200; 408.5 nm; c, 135.000; ratio: 2.07

Methyl pyropheophorbide *a* (by refluxing in acidic methanol then heating in pyridine)

Formula: $C_{34}H_{36}O_3N_4$ MW: 548.2786

Structure: $R_1$ = —H. $R_2$ = —H. $R_3$ = —$CH_3$

Abs. max. ether: 667.0 nm; c. 52.000; 409.0 nm; ratio: 2.09

10-Hydroxypheophytin *a* (by allomerization and acidification)

Formula: $C_{55}H_{74}O_6N_4$ MW: 886.5606

Structure: $R_1$ = —OH. $R_2$ = —$CO_2CH_3$. $R_3$ = —$C_{20}H_{38}$

9-Deoxo-9-hydroxypheophytin *a* (by reduction then acidification)

Formula: $C_{55}H_{71}O_5N_4$ MW: 872.5816

Structure: $R_1$ = —H. $R_2$ = —$CO_2CH_3$. $R_3$ = $C_{20}H_{38}$ —H and —OH in place of =O at C—9

Abs. max. ether: 655 nm; c. 53.000; 397 nm; c. 172.000

Pheophytin *a* amine products (by acidification)

General Formula: $C_{55}H_{71}O_5N_4$ (NR'R'')

Abs. spectra of pheophytin *a* + *n*-propylamine, ether: 662 nm; 441 nm

---

Porphyrins are formally derived from porphin by substitution of some or all of the peripheral positions with various side chains. Porphin has the following structural formula:

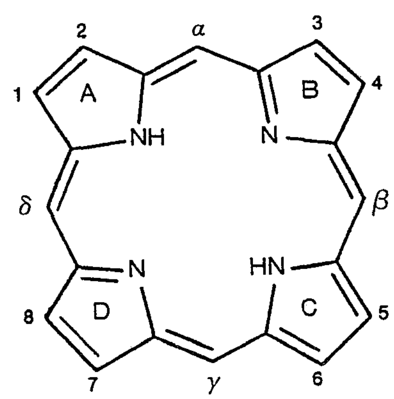

Some typical porphyrins useful in the present invention are listed in Table 4 which also gives the substituent groups and their location on the porphin structure.

TABLE 4

| Porphyrin | 1 | 2 | 3 | 4 | 5 | 6 | v | 7 | 8 |
|---|---|---|---|---|---|---|---|---|---|
| Etioporphyrin-1 | Me | Et | Me | Et | Me | Et | H | Me | Et |
| Octaethylporphyrin | Et | Et | Et | Et | Et | Et | H | Et | Et |
| Deuteroporphyrin-IX | Me | H | Me | H | Me | $p^H$ | H | $p^H$ | Me |
| Mesoporphyrin-IX | Me | Et | Me | Et | Me | $p^H$ | H | $p^H$ | Me |
| Hematoporphyrin-IX | Me | OH<br>\|<br>CH.Me | Me | OH<br>\|<br>CH.Me | Me | $p^H$ | H | $p^H$ | Me |
| Protoporphyrin-IX | Me | V | Me | V | Me | $p^H$ | H | $p^H$ | Me |
| Coproporphyrin-I | Me | $p^H$ | Me | $p^H$ | Me | $p^H$ | H | Me | $p^H$ |
| Coproporphyrin-III | Me | $p^H$ | Me | $p^H$ | Me | $p^H$ | H | $p^H$ | Me |
| Uroporphyrin-I | $A^H$ | $p^H$ | $A^H$ | $p^H$ | $A^H$ | $p^H$ | H | $A^H$ | $p^H$ |
| Uroporphyrin-III | $A^H$ | $p^H$ | $A^H$— | $p^H$ | $A^H$ | $p^H$ | H | $p^H$ | $A^H$ |
| Chlorocruoroporphyrin | Me | CHO | Me | V | Me | $p^H$ | H | $p^H$ | Me |
| Pemptoporphyrin | Me | H | Me | V | Me | $p^H$ | H | $p^H$ | Me |
| Deuteroporphyrin-IX 2,4-di-acrylic acid | Me | $Acr^H$ | Me | $Acr^H$ | Me | $p^H$ | H | $p^H$ | Me |
| 2,4-Diformyldeutero-porphyrin-IX | Me | CHO | Me | CHO | Me | $p^H$ | H | $p^H$ | Me |
| 2,4-Diacetyldeutero-porphyrin-IX | Me | Ac | Me | Ac | Me | $p^H$ | H | $p^H$ | Me |
| Deuteroporphyrin-IX 2,4-disulfonic acid | Me | $SO_3H$ | Me | $SO_3H$ | Me | $p^H$ | H | $p^H$ | Me |
| Phylloporphyrin-XV | Me | Et | Me | Et | Me | H | Me | $p^H$ | Me |
| Pyrroporphyrin-XV | Me | Et | Me | Et | Me | H | H | $p^H$ | Me |
| Rhodoporphyrin-XV | Me | Et | Me | Et | Me | $CO_2H$ | H | $p^H$ | Me |
| Phylloerythrin | Me | Et | Me | Et | Me | CO— | $CH_2$ | $p^H$ | Me |
| Desoxophylloerythrin | Me | Et | Me | Et | Me | $CH_2$— | $CH_2$ | $p^H$ | Me |
| Pheoporphyrin-$a_5$ | Me | Et | Me | Et | Me | CO— | CH<br>\|<br>$CO_2Me$ | $p^H$ | Me |

*Side-chain abbreviations:* Me = Methyl; Et = Ethyl; V = Vinyl; $p^H = CH_2CH_2CO_2H$; $A^R = CH_2CO_2R$; Ac = CO.Me; $Acr^H = CH{=}CH.CO_2H$

Table 5 lists additional porphyrins useful in the present invention and includes the substituents thereof and spectroscopic absorption data:

TABLE 5

| Porphyrin | Substituents 2 | 4 | $pK_3$[a] ±0.1 unit | Band I[b] (nm) | Soret[a] (nm) |
|---|---|---|---|---|---|
| Meso | Et | Et | 5.8 | 620 | 399 |
| Deutero | H | H | 5.5 | 618 | 398 |
| Copro | $p^{Me}$ | $p^{Me}$ | 5.5[c] | 620 | 399 |
| Proto | V | V | 4.8 | 630 | 408 |
| 2,4-Diacetyldeuterodioxime | C(Me) = NOH | C(Me) = NOH | 4.5 | 625 | 403 |
| 2-Formyl-4-vinyl-deuterooxime | CH = NOH | V | 4.4 | 635 | 415 |
| 2,4-Diformyldeuterodioxime | CH = NOH | CH = NOH | 4.3 | 639 | 414 |
| 4-Propionyldeutero | H | CO-Et | 4.2 | | 409 |
| 4-Formyldeutero | H | CHO | 3.8[c] | 640 | 413 |
| 2-Formyl-4-vinyldeutero (chlorocruoro) | CHO | V | 3.7 | 644 | |
| 2,4-Diacetyldeutero | CO-Me | CO-Me | 3.3 | 639 | 424 |
| 2,4-Dipropionyldeutero | CO-Et | CO-Et | 3.2 | | 423 |
| 4-Nitrodeutero | H | $NO_2$ | 3.2 | | 401 |
| 2-Vinyl-4-cyanodeutero | V | CN | 3.0[d] | | 413 |
| 2,4-Di-methoxycarbonyldeutero | $CO_2Me$ | $CO_2Me$ | 3.0[d] | | 423 |
| 2,4-Dibromodeutero | Br | Br | 3.0[d] | | |
| 2,4-Diformyldeutero | CHO | CHO | 3.0[c,d] | 651 | 436 |

[a] Measured at 25°C in 2.5% sodium dodecyl sulfate.
[b] In dioxan.
[c] By extrapolation from values at 20°C.
[d] ± 0.2 units, inaccuracies introduced by ionic strength variations.

Additional porphyrins useful in the present invention include 2,4-diacetyldeuterodioxime, 2-formyl-4-vinyl-deuterooxime, 2,4-diformyldeuterodioxime, 4-propionyldeutero, 4-formyldeutero, 2-formyl-4-vinyldeutero (chlorocruoro), 2,4-diacetyldeutero, 2,4-dipropionyldeutero, 4-nitrodeutero, 2-vinyl-4-cyanodeutero, 2,4-di-methoxycarbonyl-deutero, 2,4-dibromodeutero and 2,4-diformyldeutero.

Alteration products, derivatives and isomers of porphyrins are also useful in the present invention and include: chlorins, phlorins, oxophlorins, corrins, corphins, corroles and etioporphyrins. Chlorins are 7,8-dihydroporphyrins. Magnesium complexes of chlorins are the chlorophylls. Chlorins have the following structural formula:

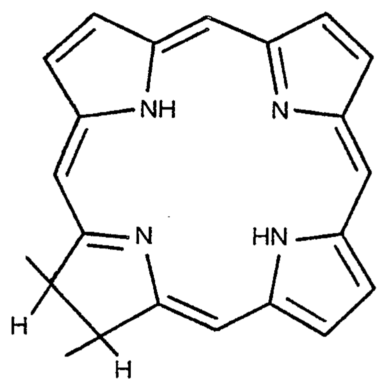

Phlorins are also dihydroporphyrins and have the following structural formula:

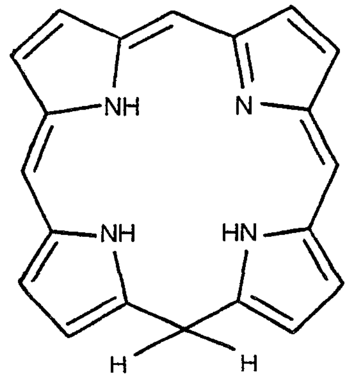

Two types of tetrahydroporphyrins are known, a- and b-tetrahydroporphyrins, and have the following structures:

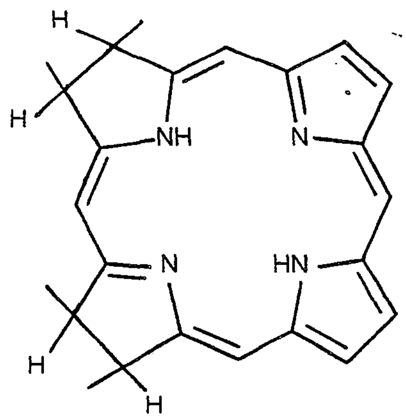

(a)

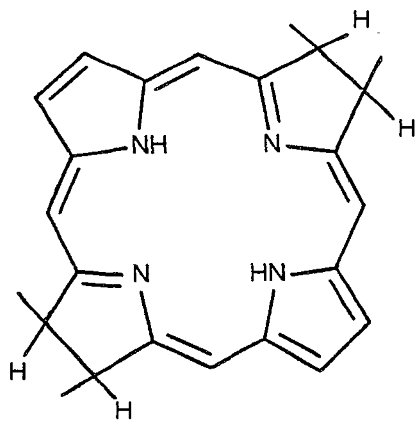

(b)

Corphins are hexahydroporphyrins and have the following structure:

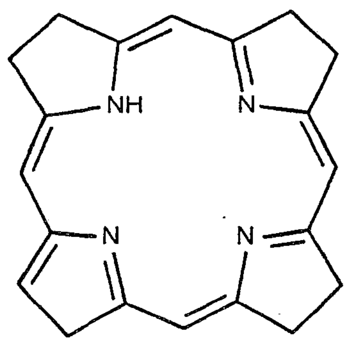

Other hexahydroporphyrins include corrins:

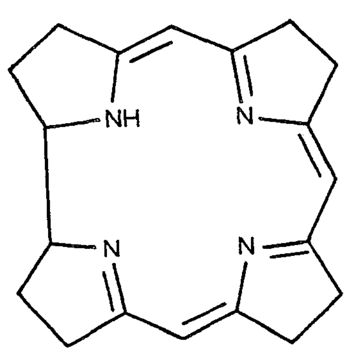

and porphyrinogens:

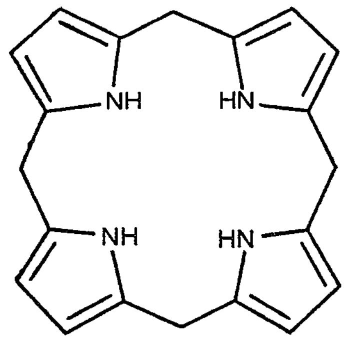

Oxophlorins are oxidized porphyrin macrocycles with oxygen functions at one to four meso-positions and have the following structures:

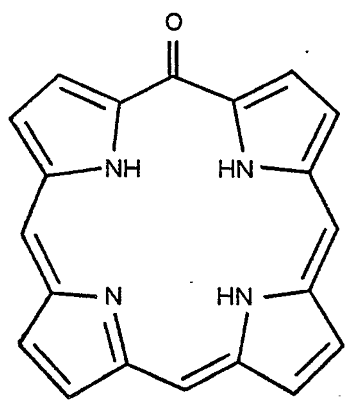

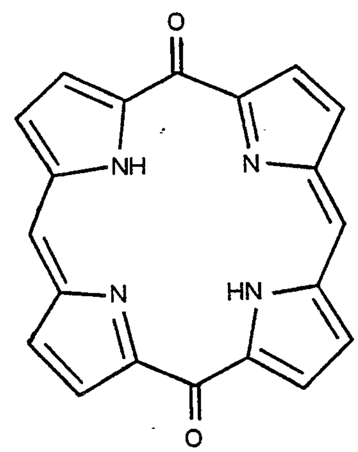

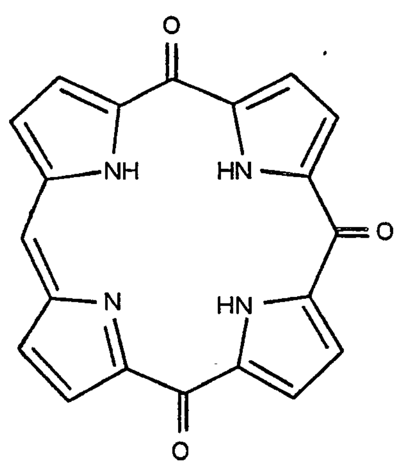

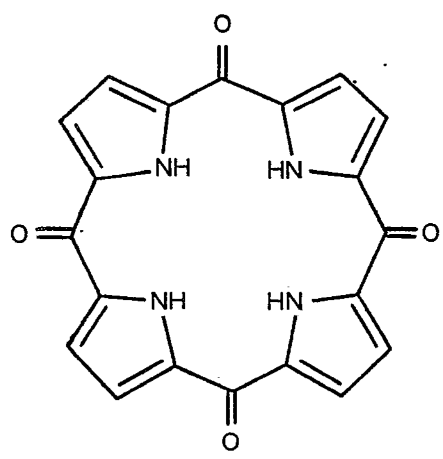

Corroles have the following structure:

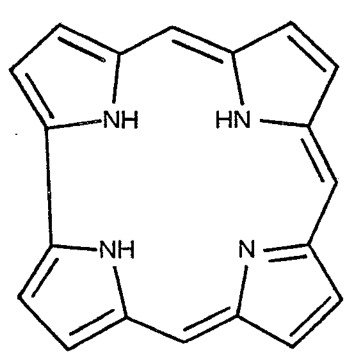

Etioporphyrins exist as four isomers and have the following structural formulas:

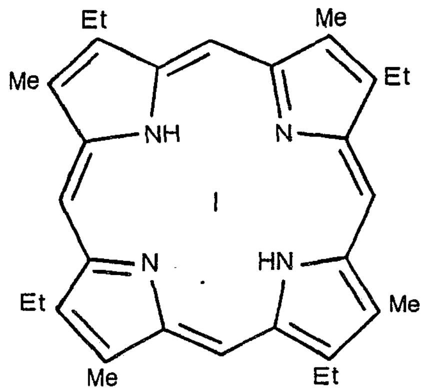
I

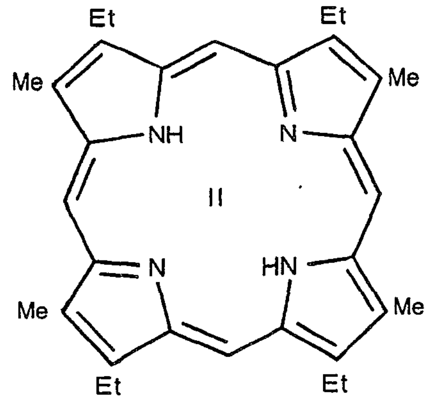
II

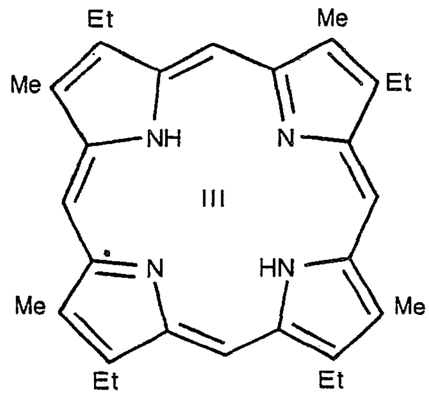
III

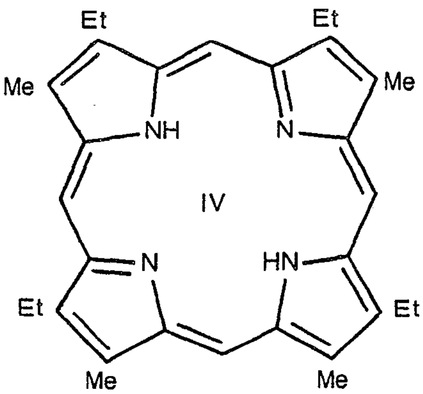
IV

All of the foregoing porphyrins have Stokes shifts in excess of 150 nanometers and are useful in the present invention. Other porphyrins and their derivatives or alteration products are also useful in the present invention as long as they have Stokes shifts of not less than 150 nanometers.

The chlorophyll or porphyrin is chemically treated so that covalent bonding can occur with the assay reagent. The method of bonding a chlorophyll or a porphyrin to the assay reagent will vary depending upon the nature of the chlorophyll or the porphyrin and the assay reagent involved; however, an amide linkage between the chlorophyll or the porphyrin and the assay reagent can be conveniently used. In the case of conjugation of bacteriochlorophyll b with an antigen, such as thyroxine, the steps involved can include saponification of the bacteriochlorophyll b to remove the phytyl chain therefrom, acylation and amide linkage to the antigen or a derivative of the antigen. An outline of the conjugation of bacteriochlorophyll b with thyroxine, triiodothyronine, thyroid stimulating hormone and digoxin is shown below:

Bacteriochlorophyll b NaOH Bacteriochlorophyll b
⟶
Heat
(SOCl$_2$/Heat
+ Thyroxine Amination with
carbodiimide or
isobutylchloro-
formate/dioxane
→ Thyroxine-Bacteriochlorophyllide b

Bacteriochlorophyll b NaOH Bacteriochlorophyllide b
⟶
Heat
(SOCl$_2$/Heat
+ triiodothyronine Amide linkage
with
carbodiimide or
isobutylchloro-
Formate/dioxane)
→ Thyroxine-Bacteriochlorophyllide b

Bacteriochlorophyll b NaOH Bacteriochorophyllide b
→
Heat

(SOCl$_2$/Heat
+ Thyroxine Amide linkage
Stimulating with
Hormone gluteraldehyde)

→ Thyroxine Stimulating Hormone —
Bacteriochlorophyllide b

Bacteriochlorophyll b NaOH Bacteriochorophyllide b
→
Heat

+ Digoxin (Succinic
anhydride/
pyridine/
carbodiimide)
→ Digoxin-Bacteriochlorophyllide b

It is within the skill of the art to determine other methods of conjugating other chlorophylls or porphyrins with other coupling agents.

The following Example is illustrative of the present invention only and is not intended to limit the scope of the invention. All temperatures are given in degrees Centigrade and all parts are parts by weight unless specifically stated.

Example

*Rhodopseudomonas viridis* G. Drews strain (ATCC 19567) was obtained from the American Type Culture Collection, Rockville, Maryland. A flask containing the following medium was prepared: sodium succinate 2.5 g, $K_2PO_4$, $MgSO_4.7H_2O$ 0.2 g, $(NH_4)_2SO_4$ 1.25 g, $CaCl_2$ 0.07 g, ferric citrate 0.003 g, EDTA 0.002 g, yeast extract 0.5 g and deionized water 1.0. The pH was adjusted to 7.0. The bacteria was added to the medium in the flask which was then tightly stoppered and placed under a ttungsten lamp. The bacteria in the flask was maintained at a temperature of 30°C for a period of five days after which time a dark green color was observed in the liquid medium, thereby indicating prolific growth of the bacteria.

Aliquots of the medium were transferred to test tubes and centrifuged at 1,000 times gravity for 15 min at 20°C. The supernatants were discarded and the bacteria cells remaining in the test tubes were resuspended with 1.0 ml of acetone. The addition of the acetone to the bacteria cells disrupted the cell membranes and extracted the bacteriochlorophyll into the solvent solution. The extracted solutions were pooled together and filtered through a 20 µm pore polyethylene filter obtained from Glasrock Products, Fairburn, Georgia. A 250 ml quantity of the filtered solution was then introduced into a rotary flash evaporator flask. The flask was rotated at 90 RPM in boiling water. The acetone was evaporated off leaving a residue of bacteriochlorophyll and other extracted products.

The residue in the flask was dissolved in petroleum ether. Then, 25 ml aliquots of the solution were pipetted onto a silica gel G thin-layer chromatographic plate and developed with petroleum ether-acetone-propanol having a ratio of 9:1:0.45. Zones containing pigments which fluoresced were observed with a long-wave ultraviolet lamp, were scraped off the plate and eluted with methanol. The thin layer chromatography extracts were injected into a high pressure liquid chromatography (HPLC) instrument obtained from Micromeritics, Inc., Norcross, Georgia. Purified samples were collected from the HPLC after the extracts had been forced through a reversed-phase octadecylsilene packed column. The collected samples were again injected into the HPLC and the fractions having sharp fluorescent peaks were collected.

The final product of the thin-layer chromatography and the HPLC was bacteriochlorophyllide b. This was confirmed by UV-Visible, Fluorescent, Infrared and Nuclear Magnetic Resonance spectroscopy.

The bacteriochlorophyllide b is then conjugated with thyroxine as described below:

Isobutylchloroformate in a solution of triethylamine and dioxan reacts with bacteriochlorophyllide b to form the compound $RCOOCO_2CH_2CH(CH_3)_2$ where RCOO— is the bacteriochlorophyllide b portion of the compound. Next thyroxine is added to form the compound RCONH—R′ where R is the bacteriochlorophyllide b portion and R′ is thyroxine as shown below.

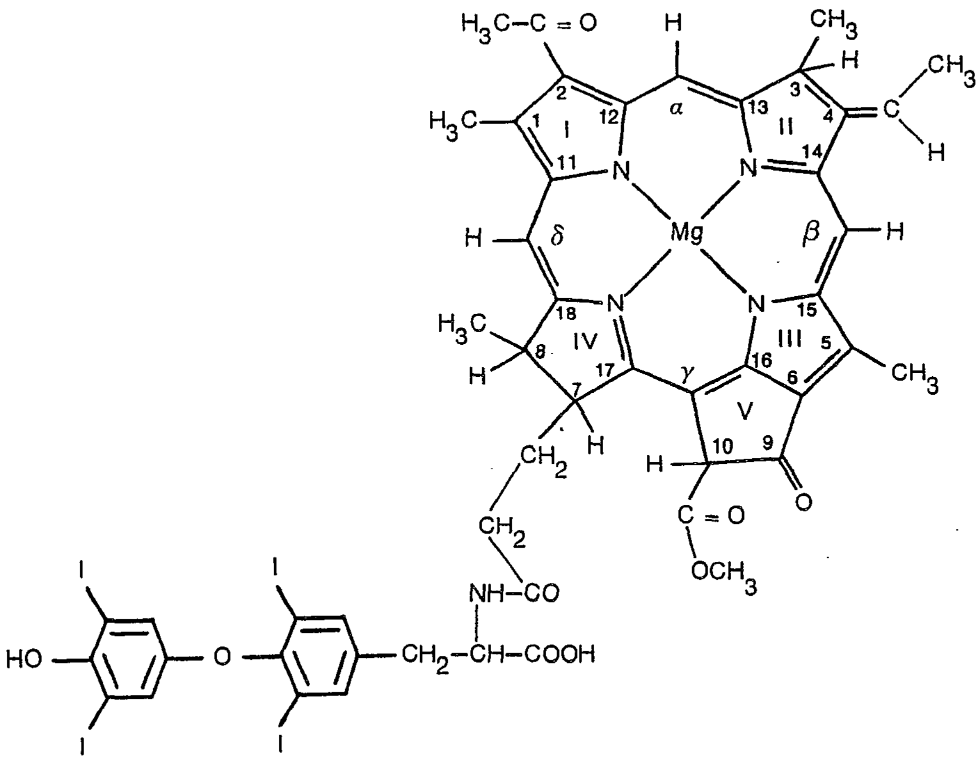

After the bacteriochlorophyllide b and thyroxine have been conjugated to provide thyroxine — bacteriochlorophyllide b, the concentration of the labelled antigen is determined using a radioimmunoassay procedure. A dose-response curve is then prepared for various dilutions of the conjugated thyroxine — bacteriochlorophyllide b to determine optimal concentration for the assay.

A whole blood sample is taken from a patient and centrifuged to separate the blood cells from the blood serum. The blood serum is then drawn off for testing.

A plurality of test tubes which have been previously coated with anti-thyroxine antibody are obtained. Such coated test tubes are commercially available and can also comprise wells or microtiter plates instead of test tubes. Conventionally, the antibody is coated in a thin layer onto the inner glass wall at the bottom of a test tube, however, the antibody may in some cases be coated or covalently bonded to a test tube made of polystyrene or polypropylene. 2 to 50 µl of the patient's blood serum is then added to the coated test tube along with a series of known quantities of thyroxine (calibrators) and serum controls in additional tubes. For example, solutions containing 0, 2, 4, 8, 10, 12 and 14 µg of thyroxine per dl of solution are prepared and added to the coated test tubes. To each standard solution is added a known amount, e.g. 1 to 3 ml, of the thyroxine-bacteriochlorophyllide b solution prepared above. The fluorescent material is in a buffer solution such as sodium barbital buffer, sodium carbonate-bicarbonate buffer, tris buffer (tris(hydroxymethyl)-amino-methan), phosphate buffered saline or sodium acetate buffer. The amount of buffer added to the fluorescent material is such that the concentration of the fluorescent material is approximately the same as the concentration of the standard to which it is added. A known amount of the fluorescent material in buffer solution is also added to the test tube containing the patient blood serum.

The standard and patient test tubes are then mixed in a vortexer and incubated at a temperature of between 25° and 37° for a period of 15 min to 3 h; preferably one hour. The liquid in the test tubes is then aspirated or decanted off and the test tubes are rinsed with deionized water or buffer solution. A solvent solution, such as acetone, methanol or acetonitrile, is added to the test tube to extract off the fluorescent material from the test tube wall. Each solvent solution for the standards and patient samples, which contains both marked and unmarked antigen-antibody complexes, is exposed to radiation of a wavelength sufficient to excite the fluorescent material and the emitted radiation therefrom is measured. The concentration of the tyroxine in the patient sample can then be determined.

An apparatus to perform the assays described hereinabove is shown in the drawing figures wherein like numerals reference like parts. Fig. 1 is a pictorial diagram of the first embodiment of the apparatus. The multiplexing and computing apparatus is a conventional stand-alone microcomputer unit contained in a housing 10. A pair of mini-floppy disk drives 11a and 11b are included for storage of patient records and to store other programs. The microcomputer also includes a conventional keyboard 15. The apparatus uses a desk top main frame including CRT display 12, disk drives 11 and keyboard 15 manufactured under the name EXORSET 30 which is currently manufactured by Motorola Semiconductor Products, Inc., of Phoenix, Arizona. This embodiment uses a Motorola-type M68MM19 one board microcomputer inserted on the main frame shown in Fig. 1. It will be appreciated by those skilled in the art that the M68MM19 is a one

board microcomputer using an MC6809 central processing unit interfacing to a sixteen bit system address bus, an eight bit system data bus and a fifteen bit system control bus.

The one board microcomputer also includes buffered eight and four bit parallel input/output ports and a serial input/output port which may be configured for either the RS232C or RS422 standards promulgated by the Electronics Institute of America. The microcomputer further includes direct plug-in sockets for up to 16 K of read only memory.

It should be understood that the read only memory of the microcomputer used in this embodiment is programmed to calculate both dose response curves and the patient antigen levels as described herein. The calculations of these parameters are known to those skilled in the art and the routines for calculating.

Connected to microcomputer 10 shown in Fig. 1 is an eight by twelve array 16 of photodetectors upon which is placed an eight by twelve microtitration multi well plate 17.

Each photo sensor of the array of photosensors 16 is characterized by having a frequency response within the following parameters. The photodetectors must exhibit a substantial response to light having a wavelength of 680 nanometers. Using the response 680 nanometers as a 0 dB reference, the electrical output of the photodetector must be at least 10 dB down in response to light at 560 nanometers. This requirement assumes equal luminous flux impinging on the photodetector at both wavelengths. This requirement follows from the characteristics of the fluorescent labels described hereinabove and the fact that the apparatus is intended to be usable in an environment where the wells of microtitration plate 17 may contain serum substances which autofluoresce at wavelengths in the range from 500 to 520 nanometers. In this embodiment, each of the photodetectors of array 16 is a type HEDS—1000 currently manufactured by Hewlett-Packard Incorporated, Optoelectronics Division, of Palo Alto, California. A frequency response curve of the voltage output of this particular optodetector is shown in Fig. 5 under conditions of constant luminus flux.

Thus it will be appreciated that the type HEDS—1000 meets the criteria set forth above for photodetectors used in the apparatus.

Also shown in Fig. 1 is the remainder of the apparatus. Another eight by twelve array of elements is shown as 19. Each of the elements of array 19 is a conventional termination for one of a group of ninety-six fiber optic links 20 which carries light from a source of ultraviolet light 21. It is to be understood that ultraviolet light 21 is a conventional fluorescent ultraviolet light and comprises a source of excitation radiation.

Fiberoptics 20 comprise a plurality of light conducting fibers, each being arranged to accept the excitation radiation from ultraviolet source 21 at one end of the fiber and transmitting it to the termination at terminal array 19. It should be understood that each element of terminal array 19 is a polished end of one of the fibers of fiber optic cable 20. It should further be appreciated from inspection of Fig. 1 that each fiber of cable 20 is joined to terminal array 19 in a way which causes each of the fibers to be joined to one of terminals in an array defining a predetermined spaced relationship to the remainder of the fibers.

In use, it will be appreciated that terminal block 19 is set over microtitration plate 17 so that one element of each of arrays 16, 17 and 19 are lined up in a manner which places their approximate geometric centers on the same line.

Thus it will be appreciated that when the arrangement is connected, and each well of microtitration plate 17 contains a sample prepared as described hereinabove, each of the conductors of cable 18 will carry a voltage proportional to the luminous flux of approximately 680 nanometers impinging on the element of photodetector array 16 to which the particular conductor is connected.

A block diagram of the apparatus is shown in Fig. 2. Ultraviolet source 21 is shown as connected through fiber optic cable 20, only three fibers of which are shown, to a plurality of terminations 22 as previously described. Microtitration well array 17 is set on top of photodetector array 16, the outputs of which are coupled by cable 18 to multiplexer and analog to digital converter block 25. Multiplexer and analog to digital block 25 is connected for bidirectional communication via link 26 to M68MM19 computer board 28 described hereinabove.

Keyboard 15 provides input to one board computer 28 which communicates bidirectionally with the aforementioned disk storage shown as 11′ in Fig. 2.

Also shown in Fig. 2 is a printer 29 used for providing hard copies of the results of the assays.

Fig. 3 shows a second embodiment of an apparatus to be used with the present invention which will be understood to be a less expensive, simpler arrangement for performing the tests one at a time. It will be apparent that in using the apparatus of the second embodiment, it will be necessary to perform calculations for the dose response curve externally.

As may be seen from Fig. 3, the second embodiment comprises an ultraviolet source 21′ which illuminates a microtiter well (or a curvette) 30 under which placed a photodetector 31. It may be seen from Fig. 3 that ultraviolet source 21, microtiter well 30, and photodetector 31, all lie substantially on longitudinal axis 37 shown in Fig. 3. The output of photodetector 31 is provided to level adjustment amplifier 32, the output of which is provided to an integrated circuit digital multimeter 35.

In this embodiment, digital multimeter circuit 35 is embodied by an ADD3501 digital multimeter chip currently manufactured by National Semiconductor Corporation of Santa Clara, California.

The output of the DMM chip 35 is connected to a three and one-half digit seven segment display 36 so as to provide a numeral indication of the amount of luminous flux at the predetermined wavelength (preferably 680 nanometers) emitted by the substance contained in microtiter well 30. It is of course

apparent that level adjust amplifier 32 should be adjusted to provide an output appropriately scaled to suit three and one-half digit seven segment output 36.

Fig. 4 shows an embodiment of multiplexer and analog to digital converter block 25 together with an exemplary microtitration well plate 17. As shown in Fig. 4, ultraviolet lamp 21 is connected by fiber optic cable 20 having ninety-six elements to the terminal block array 19. An exemplary fiber optic element 46 is shown as connected to one of polished terminal connectors 22 which will be understood to be one of such terminations of array 19 shown in Fig. 1.

Enclosed in a box identified as 39 is one of the photodetectors of photodetector array 16. The electrical output of which appears on line 18′ which will be understood to be one conductor of cable 18 shown in Figs. 1 and 2.

The output from line 18′ is connected as one input to sixteen channel multiplexer 41c, the other fifteen inputs of which are designated as 40c and will be understood to be connected to conductors of cable 18. Similarly, sixteen additional channels are provided by multiplexers 41a and 41b. The inputs to these multiplexers are 40a and 40b, respectively, and also are connected to cable 18. Since multiplexer block 25 shown in Fig. 4 has a capacity of thirty-two channels, it will be readily appreciated that the arrangement shown as 25 is a typical one of three identical such boards used in the preferred embodiment to provide the ability to multiplex, one at a time, the outputs of all ninety-six elements of photodetector array 16.

Multiplexer board 25 comprises a programmable gain amplifier 42 which amplifies the outputs from multiplexers 41a—41c. The output of amplifier 42 is provided to sample and hold gate 47, the output of which appears on line 48 as the analog input to twelve bit analog to digital converter 49. The timing and conversion is controlled by conversion control block 50.

The output of A/D converter 49 is provided on line 51 to eight tristate buffers 52 which interface board 25 with the eight bit data bus 55 connected to the aforementioned single board microcomputer. An eight bit bus 56 carries data words, when appropriately addressed, to mode control block 57 and gain and multiplexer control 58. Mode control block 57 controls the particular sequence of signals which must appear on the address and data buses of the system to operate conversion controller 50 to cause the analog to digital conversions to take place.

An address buffer and board select decoder switches are designated as 59 in Fig. 4. While no connections are shown, it should be understood that outputs from this block control the particular elements on board 25 into which data is written or from which it is read during any particular input/output cycle from the single board computer.

It should be appreciated by those skilled in the art that the form of board 25 shown in Fig. 4 is available as a module as model No. M68MM15A currently manufactured by Motorola Semiconductor Products, Inc. of Phoenix, Arizona.

In examining the typical arrangement of polished terminal 22, microtiter well 38, and photodetector 39, it should be noted that at the point of termination 22 of fiberoptic element 46, fiberoptic 46 is characterized by longitudinal axis 45. As shown in the drawing, photodetector 39 lies on longitudinal axis 45 and microtiter well 22 is interposed between termination 22 and photodetector 39 so that longitudinal axis 49 passes substantially through the center of the microtiter well.

**Claims**

1. A fluorescent labelled reagent comprising an assay reagent conjugated with a chlorophyll having a Stokes shift of not less than 150 nanometers.

2. A fluorescent labelled reagent comprising an assay reagent conjugated with a porphyrin having a Stokes shift of not less than 150 nanometers.

3. The fluorescent labelled reagent of Claims 1 or 2 wherein the assay reagent is selected from antibodies, antigens, hormones, virus particles, haptens, bacterial components, drugs, monoclonal antibodies, anti-antibodies, immunoglobulins and proteins.

4. The fluorescent labelled reagent of Claim 1 wherein the chlorophyll is selected from chlorophyll a, chlorophyll b, chlorophyll $c_1$, chlorophyll $c_2$, chlorophyll d, protochlorophyll and chlorobium chlorophyll.

5. The fluorescent labelled reagent of Claim 1 wherein the chlorophyll is selected from bacteriochlorophyll $a_p$, bacteriochlorophyll $a_{gg}$, and bacteriochlorophyll b.

6. The fluorescent labelled reagent of Claim 2 wherein the porphyrin is selected from chlorins, phlorins, oxophlorines, corins, corphins, corroles and etioporphyrins.

7. The fluorescent labelled reagent of Claim 2 wherein the porphrin is selected from etioporphyrin-I, octaethylporphyrin, deuteroporphyrin-IX, mesoporphyrin-IX, hematoporphyrin-IX, protoporphyrin-IX, coproporphyrin-I, coproporphyrin-III, uroporphyrin-I, uroporphyrin-III, chlorocruoroporphyrin, pemptoporphyrin, deuteroporphyrin-IX, 2,4-di-acrylic acid, 2,4-diformyldeuteroporphyrin-IX, 2,4-diacetyldeuteroporphyrin-IX, deuteroporphyrin-IX, 2,4-disulfonic acid, phylloporphyrin-XV, pyrroporphyrin-XV, rhodoporphyrin-XV, phylloerythrin, desoxophylloerythrin, pheoporphyrin-$a_5$, uroporphyrin-III, pheoporphyrin-$a_5$, pheophytin-a, pheophorbide-a, pheophorbide-b, mesopheophorbide-a, chlorin-$e_6$, mesochlorin-$e_6$, and rhodin-$g_7$, 2,4-diacetyldeuterodioxime-, 2-formyl-4-vinyl-deuterooxime-, 2,4-diformyldeuterodioxime-, 4-Propionyldeutero-, 4-formyldeutero-, 2-formyl-4-vinyldeutero-, 2,4-diacetyldeutero-, 2,4-dipropionyldeutero-, 4-nitrodeutero-, 2-vinyl-4-cyanodeutero-, 2,4-di-methoxycarbonyldeutero-, 2,4-dibromodeutero- and 2,4-diformyldeutero porphyrins.

8. The fluorescent labelled reagent of Claims 1 or 2, wherein the assay reagent is selected from thyroxine, triiodothyronine, thyroid stimulating hormone, thyroxine binding globulins, thyrotropin releasing hormone, digoxin, gentamicin, tobramycin, phenytoin, theophyllin, tetracycline, Hepatitis B surface antigen, Hepatitis B core antigen, Hepatitis A antigen, carcinoembryonic antigen, prostatic acid phosphatase and human chorionic gonadotropin.

9. A method of preparing a fluorescent labelled reagent comprising the steps of conjugating an assay reagent with a chlorphyll according one or more of claims 1, 3, 4, 5 and 8.

10. A method of preparing a fluorescent labelled reagent comprising the steps of conjugating an assay reagent with a porphyrin according one or more of claims 2, 6, 7 and 8.

11. A method of analyzing a sample by immunofluorometric techniques comprising the steps of:

irradiating an antigen-antibody complex labelled with a chlorophyll having a Stokes shift of not less than 150 nanometers with an excitation radiation having a frequency band sufficient to excite said chlorophyll; and

detecting and measuring emissive radiation from said chlorophyll labelled antigen-antibody complex.

12. A method of analyzing a sample by immunofluorometric techniques comprising the steps of:

irradiating an antigen-antibody complex labelled with a porphyrin having a Stokes shift of not less than 150 nanometers with an excitation radiation having a frequency band sufficient to excite said porphyrin; and

detecting and measuring emissive radiation from said porphyrin labelled antigen-antibody couple.

13. The method of claims 11 or 12 wherein the chlorophyll is selected from chlorphyll a, chlorophyll b, chlorophyll $c_1$, chlorophyll $c_2$, chlorophyll d, protochlorophyll and chlorobium chlorophyll.

14. The method of claims 11 or 12 wherein said chlorophyll is selected from bacteriochlorophyll $a_p$, bacteriochlorophyll $a_{gg}$ and bacteriochlorophyll b.

15. The method of claims 11 or 12 wherein the porphyrin is selected from chlorins, phlorins, oxophlorines, corins, corphins, corroles and etioporphyrins.

16. The method of claims 11 or 12 wherein said porphyrin is selected from etioporphyrin-I, octaethylporphyrin, deuteroporphyrin-IX, mesoporphyrin-IX, hematoporphyrin-IX, protoporphyrin-IX, coproporphyrin-I, coproporphyrin-III, uroporphyrin-I, uroporphyrin-III, chlorocruoroporphyrin, pemptoporphyrin, deuteroporphyrin-IX, 2,4-di-acrylic acid, 2,4-diformyldeuteroporphyrin-IX, 2,4-diacetyldeuteroporphyrin-IX, deuteroporphyrin-IX 2,4-disulfonic acid, phylloporphyrin-XV, pyrroporphyrin-XV, rhodoporphyrin-XV, phylloerythrin, desoxophylloerythrin, pheoporphyrin-$a_5$, uroporphyrin-III, pheoporphyrin-$a_5$, pheophytin-a, pheophorbide-a, pheophorbide-b, mesopheophorbide-a, chlorin-$e_6$, mesochlorin-$e_6$, rhodin-$g_7$, 2,4-diacetyldeuterodioxime-, 2-formyl-4-vinyl-deuterooxime-, 2,4-diformyldeuterodioxime-, 4-propionyldeutero-, 4-formyldeutero-, 2-formyl-4-vinyldeutero-, 2,4-diacetyldeutero-, 2,4-dipropionyldeutero-, 4-nitrodeutero-, 2-vinyl-4-cyanodeutero-, 2,4-di-methoxycarbonyldeutero-, 2,4-dibromodeutero- and 2,4-diformyldeutero-porphyrins.

**Patentansprüche**

1. Fluoreszierend markiertes Reagens, gekennzeichnet durch ein mit einem Chlorophyll mit einer Stokes-Verschiebung von nicht weniger als 150 nm konjugiertes Testreagens.

2. Fluoreszierend markiertes Reagens, gekennzeichnet durch ein mit einem Porphyrin mit einer Stokes-Verschiebung von nicht weniger als 150 nm konjugiertes Testreagens.

3. Fluoreszierend markiertes Reagens nach Anspruch 1 oder 2, dadurch gekennzeichnet, daß das Testreagens aus Antikörpern, Antigenen, Hormonen, Viruspartikeln, haptenen, Bakterienkomponenten, Drogen, monoklonalen Antikörpern, Anti-Antikörpern, Immunglobulinen und Proteinen gewählt ist.

4. Fluoreszierend markiertes Reagens nach Anspruch 1, dadurch gekennzeichnet, daß das Chlorophyll aus Chlorophyll a, Chlorophyll b, Chlorophyll $c_1$, Chlorophyll $c_2$, Chlorophyll d, Protochlorophyll und Chlorobiumchlorophyll gewählt ist.

5. Fluoreszierend markiertes Reagens nach Anspruch 1, dadurch gekennzeichnet, daß das Chlorophyll aus Bacteriochlorophyll $a_p$, Bacteriochlorophyll $a_{gg}$ und Bacteriochlorophyll b gewählt ist.

6. Fluoreszierend markiertes Reagens nach Anspruch 2, dadurch gekennzeichnet, daß das Porphyrin aus Chlorinen, Phlorinen, Exophlorinen, Corrinen, Corphinen, Corrolen und Etioporphyrinen gewählt ist.

7. Fluoreszierend markiertes Reagens nach Anspruch 2, dadurch gekennzeichnet, daß das Porphyrin gewählt ist aus Etioporphyrin-I, Octäthylporphyrin, Deuteroporphyrin-IX, Mesoporphyrin-IX, Hämatoporphyrin-IX, Protoporphyrin-IX, Coproporphyrin-I, Coproporphyrin-III, Uroporphyrin-I, Chlorcruorporphyrin, Pemtoporphyrin, Deuteroporphyrin-IX-2,4-di-acrylsäure, 2,4-Diformyldeuteroporphyrin-IX, 2,4-diacetyldeuteroporphyrin-IX, Deuteroporphyrin-IX-2,4-disulfonsäure, Phylloporphyrin-XV, Pyrroporphyrin-XV, Rhodoporphyrin-XV, Phylloerythrin, Desoxophylloerythrin, Pheoporphyrin-$a_5$, Uroporphyrin-III, Pheophytin-a, Pheophorbid-a, Pheophorbid-b, Mesopheophorbid-a, Chlorin-$e_6$, Mesochlorin-$e_6$ und Rhodin-$g_7$, 2,4-Diacetyldeuterodioxim, 2-Formyl-4-vinyl-deuterooxim-, 2,4-Diformyldeuterodioxim-, 4-Propionyldeutero-, 4-Formyldeutero-, 2-Formyl-4-vinyldeutero-, 2,4-Diacetyldeutero-, 2,4-Dipropionyldeutero-, 4-Nitrodeutero-, 2-Vinyl-4-cyandeutero-, 2,4-Di-methoxycarbonyldeutero-, 2,4-Dibromdeutero- und 2,4-Diformyldeuteroporphyrin.

8. Fluoreszierend markiertes Reagens nach Anspruch 1 oder 2, dadurch gekennzeichnet, daß das Testreagens gewählt ist aus Thyroxin, Trijodthyronin, thyroidstimulierendem Hormon, thyroxin-bindendem Globulin, Thyrotropin freisetzendem Hormon, Digoxin, Gentamicin, Tobramycin, Phenytoin, Theophyllin, Tetracyclin, Hepatitis-B-Oberflächenantigen, Hepatitis-B-Kernantigen, Hepatitis-A-Antigen, carcinoembryonischem Antigen, saurer Phosphatase der Prostata und menschlichem Choriongonadotropin.

9. Verfahren zur Herstellung eines fluoreszierend markierten Reagens, dadurch gekennzeichnet, daß ein Testreagens mit einem Chlorophyll nach einem oder mehreren der Ansprüche 1, 3, 4, 5 und 8 konjugiert wird.

10. Verfahren zur Herstellung eines fluoreszierend markierten Reagens, dadurch gekennzeichnet, daß ein Testreagens mit einem Porphyrin nach einem oder mehreren der Ansprüche 2, 6, 7 und 8 konjugiert wird.

11. Verfahren zur immuno-fluorometrischen Analyse einer Probe, dadurch gekennzeichnet, daß ein mit einem Chlorophyll mit einer Stokes-Verschiebung von nicht weniger als 150 nm markierter Antigen-Antikörper-Komplex mit erregender Strahlung in einem Frequenzband bestrahlt wird, das zur Erregung des Chlorophylls ausreicht, und daß die von dem chlorophyllmarkierten Antigen-Antikörper-Komplex ausgehende Strahlung erfaßt und gemessen wird.

12. Verfahren zur immuno-fluorometrischen Analyse einer probe, dadurch gekennzeichnet, daß ein mit einem Porphyrin mit einer Stokes-Verschiebung von nicht weniger als 150 nm markierter Antigen-Antikörper-Komplex mit einer erregenden Strahlung in einem Frequenzband bestrahlt wird, das zur Erregung des Porphyrins ausreicht, und daß die von dem porphyrinmarkierten Antigen-Antikörper-Komplex ausgehende Strahlung erfaßt und gemessen wird.

13. Verfahren nach Anspruch 11 oder 12, dadurch gekennzeichnet, daß das Chlorophyll aus Chlorphyll a, Chlorophyll b, Chlorophyll $c_1$, Chlorophyll $c_2$, Chlorophyll d, Protochlorophyll und Chlorobiumchlorophyll gewählt wird.

14. Verfahren nach Anspruch 11 oder 12, dadurch gekennzeichnet, daß das Chlorophyll aus Bacteriochlorophyll $a_p$, Bacteriochlorophyll $a_{gg}$ und Bacteriochlorophyll b gewählt wird.

15. Verfahren nach Anspruch 11 oder 12, dadurch gekennzeichnet, daß das Porphyrin aus Chlorinen, Phlorinen, Oxophlorinen, Corrinen, Corphinen, Corrolen und Etioporphyrinen gewählt wird.

16. Verfahren nach Anspruch 11 oder 12, dadurch gekennzeichnet, daß das Porphyrin aus Etioporphyrin-I, Octäthylporphyrin, Deuteroporphyrin-IX, Mesoporphyrin-IX, Hämatoporphyrin-IX, Protoporphyrin-IX, Corpoporphyrin-I, Coproporphyrin-III, Uroporphyrin-I, Chlorcuorporphyrin, Pemptòporphyrin, Deuteroporphyrin-IX-2,4-di-acrylsäure, 2,4-Diformyldeuteroporphyrin-IX, 2,4-Diacetyldeuteroporphyrin-IX, Deuteroporphyrin-IX-2,4-disulfonsäure, Phyolloporphyrin-XV, Pyrroporphyrin-XV, Rhodoporphyrin-XV, Phylloerythrin, Desoxophylloerythrin, Pheoporphyrin-$a_5$, Uroporphyrin-III, Pheophytin-a, Pheophorbid-a, Pheophorbid-b, Mesopheophorbid-a, Chlorin-$e_6$, Mesochlorin-$e_6$, Rhodin-$g_7$, 2,4-Diacetyldeuterodioxim-, 2-Formyl-4-vinyl-deuterooxim-, 2,4-Diformyldeuterodioxim-, 4-Propionyldeutero-, 4-Formyldeutero-, 2-Formyl-4-vinyldeutero-, 2,4-Diacetyldeutero-, 2,4-Dipropionyldeutero-, 4-Nitrodeutero-, 2-Vinyl-4-cyandeutero-, 2,4-Di-methoxycarbonyldeutero-, 2,4-Dibromdeutero- und 2,4-Diformyldeuteroporphyrin gewählt wird.

**Revendications**

1. Réactif fluorescent marqué comprenant un réactif d'analyse conjugué avec une chlorophylle présentant un décalage de Stokes qui n'est pas inférieur à 150 nanomètres.

2. Réactif fluorescent marqué comprenant un réactif d'analyse conjugué avec une porphyrine présentant un décalage de Stokes qui n'est pas inférieur à 150 nanomètres.

3. Réactif fluorescent marqué suivant l'une des revendications 1 et 2, caractérisé en ce que le réactif d'analyse est choisi par mi les anticorps, antigènes, hormones, particules virales, haptènes, composants bactériens, drogues, anticorps monocloniques, anti-anticorps, immunoglobulines et protéines.

4. Réactif fluorescent marqué suivant la revendication 1, caractérisé en ce que la chlorophylle est choisie par mi les chlorophylles *a*, chlorophylle *b*, chlorophylle $c_1$, chlorophylle $c_2$, chlorophylle *d*, protochlorophylle et chlorobiumchlorophylle.

5. Réactif fluorescent marqué suivant la revendication 1, caractérisé en ce que la chlorophylle est choisie parmi les bactériochlorophylle $a_p$, bactériochlorophylle $a_{gg}$ et bactériochlorophylle *b*.

6. Réactif fluorescent marqué suivant la revendication 2, caractérisé en ce que la porphyrine est choisie parmi les chlorines, phlorines, oxophlorines, corrines, corphines, corroles et étioporphyrines.

7. Réactif fluorescent marqué suivant la revendication 2, caractérisé en ce que la porphyrine est choisie parmi les étioporphyrine-I, octaéthylporphyrine, deutéroporphyrine-IX, mésoporphyrine-IX, hématoporphyrine-IX, protoporphyrine-IX, coproporphyrine-I, coproporphyrine-III, uroporphyrine-I, uroporphyrine-III, chlorocruoroporphyrine, pemptoporphyrine, acide deutéroporphyrine-IX-2,4di-acrylique, 2,4-diformyldeutéroporphyrine-IX, 2,4-diacétyldeuteroporphyrine-IX, acide deutéroporphyrine-IX, 2,4-disulfonique, phylloporphyrine-XV, pyrroporphyrine-XV, rhodoporphyrine-XV, phylloérythrine, désoxophylloérythrine, phéopòrphyrine-$a_5$, uroporphyrine-III, phéoporphyrine-$a_5$, phéophytine-a,

phéophorbide-a, phéophorbide-b, mésophéophorbide-a, chlorine-$e_6$, mésochlorine-$e_6$ et rhodine-$g_7$, 2,4-diacéthyldeutérodioxime-, 2-formyl-4-vinyl-deutéro-oxime, 2,4-diformyldeutérodioxime-, 4-propionyldeutéro-, 4-formyldeutéro-, 2-formyl-4-vinyl-deutéro-, 2,4-diacétyldeutéro-, 2,4-dipropionyldeutéro-4-nitrodeutéro-, 2-vinyl-4-cyanodeutéro-, 2,4-di-méthoxy-carbonyldeutéro-, 2,4-dibromodeutéro- et 2,4-diformyldeutéro-porphyrine.

8. Réactif fluorescent marqué suivant l'une des revendications 1 et 2, caractérisé en ce que le réactif d'analyse est choisi parmi les thyroxine, triiodothyronine, hormone stimulant la thyroïde, les globulines se liant à la thyroxine, l'hormone libérant la thyrotropine, les digoxine, gentamycine, tobramycine, phénytoïne, theophylline, tétracycline, antigène de surface de l'hépatite B, antigène de noyau de l'hépatite B, antigène de l'hépatite A, antigène carcinoembryonique, phosphatase de l'acide prostatique et gonadotropine chorionique humaine.

9. Procédé pour préparer un réactif fluorescent marqué, caractérisé en ce qu'il comprend les étapes de conjugaison d'un réactif d'analyse avec une chlorophylle suivant une ou plusieurs des revendications 1, 3, 4, 5 et 8.

10. Procédé pour la préparation d'un réactif fluorescent marqué, caractérisé en ce qu'il comprend les étapes de conjugaison d'un réactif d'analyse avec une porphyrine suivant une ou plusieurs des revendications 2, 6, 7 et 8.

11. Procédé pour analyser un échantillon par des méthodes immunofluorométriques, caractérisé en ce qu'il comprend les étapes d'irradiation d'un complexe antigène-anticorps marqué avec une chlorophylle présentant un déplacement de Stokes qui ne soit pas inférieur à 150 nm avec une radiation d'excitation ayant une bande de fréquence suffisante pour exciter cette chlorophylle, et une étape de détection et de mesure de l'émission de radiation par le complexe antigène-anticorps marqué par la chlorophylle.

12. Procédé pour l'analyse d'un échantillon par des techniques immunofluorométriques, caractérisé en ce qu'il comprend les étapes de: irradiation d'un complexe antigène-anticorps marqué avec une porphyrine présentant un décalage de Stokes qui n'est pas inférieur à 150 nm avec une radiation d'excitation ayant une bande de fréquence suffisante pour exciter cette porphyrine, et la détection et la mesure de la radiation émise par le couple antigène-anticorps marqué par la porphyrine.

13. Procédé suivant les revendications 11 et 12, caractérisé en ce que la chlorophylle est choisie par mi les chlorophylle *a*, chlorophylle *b*, chlorophylle $c_1$, chlorophylle $c_2$, chlorophylle *d*, protochlorphylle et chlorobiumchlorophylle.

14. Procédé suivant les revendications 11 et 12, caractérisé en ce que la chlorophylle est choisie par mi les bactériochlorophylle $a_p$, bactériochlorophylle $a_{gg}$ et bactériochlorophylle *b*.

15. Procédé suivant les revendications 11 et 12, caractérisé en ce que la porphyrine est choisie parmi les chlorines, phlorines, oxophlorines, corrines, corphines, corroles et étioporphyrines.

16. Procédé suivant les revendications 11 et 12, caractérisé en ce que la porphyrine est choisie parmi les étioporphyrine-I, octaéthylporphyrine, deutéroporphyrine-IX, mésoporphyrine-IX, hématoporphyrine-IX, protoporphyrine-IX, coproporphyrine-I, coproporphyrine-III, uroporphyrine-I, uroporphyrine-III, chlorocruoroporphyrine, pemptoporphyrine, acide deutéroporphyrine-IX-2,4-di-acrylique, 2,4-diformyldeutéroporphyrine-IX, 2,4-diacétyldeuteroporphyrine-IX, acide deutéroporphyrine-IX, 2,4-disulfonique, phylloporphyrine-XV, pyrroporphyrine-XV, rhodoporphyrine-XV, phylloérythrine, désoxophylloérythrine, phéoporphyrine-$a_5$, uroporphyrine-III, phéoporphyrine-$a_5$, phéophytine-a, phéophorbide-a, phéophorbide-b, mésophéophorbide-a, chlorine-$e_6$, mésochlorine-$e_6$ et rhodine-$g_7$, 2,4-diacéthyldeutérodioxime-, 2-formyl-4-vinyl-deutéro-oxime-, 2,4-diformyldeutérodioxime-, 4-propionyldeutéro-, 4-formyldeutéro-, 2-formyl-4-vinyl-deutéro-, 2,4-diacétyldeutéro-, 2,4-dipropionyldeutéro-, 4-nitrodeutéro-, 2-vinyl-4-cyanodeutéro-, 2,4-di-méthoxycarbonyldeutéro-, 2,4-dibromodeutéro-, et 2,4-diformyldeutéroporphyrine.

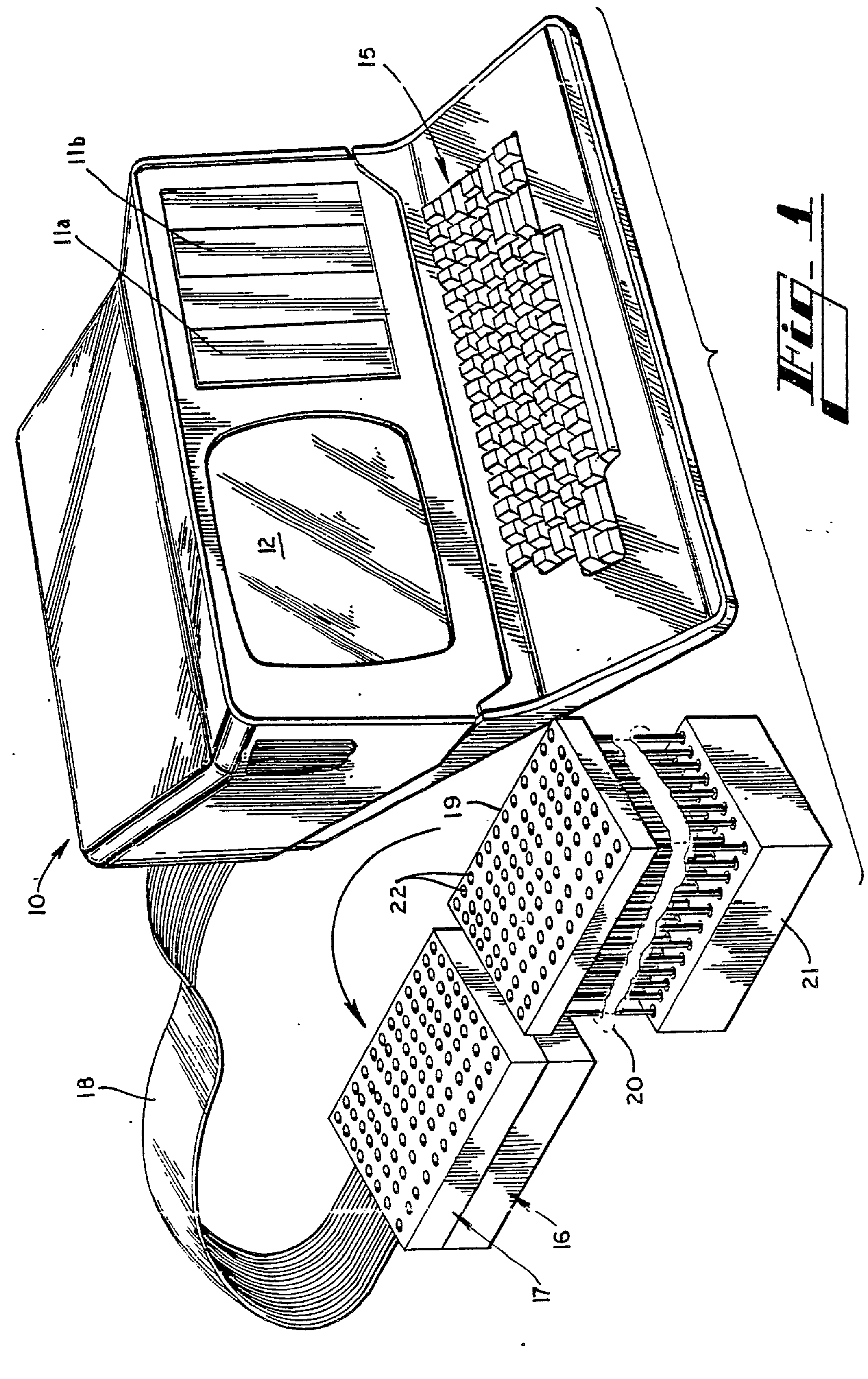


Fig. 1

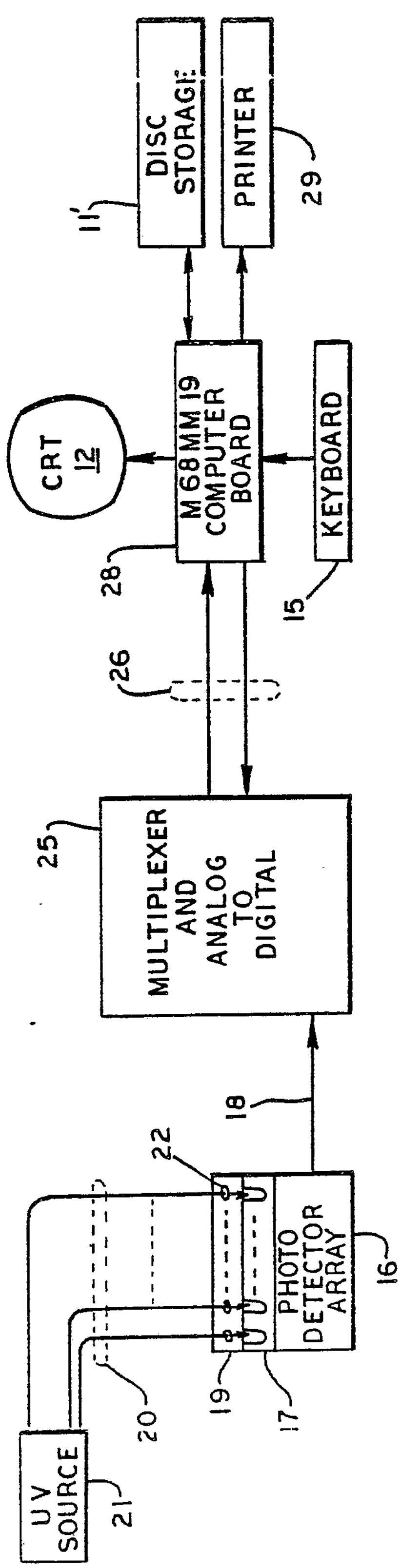


Fig_2

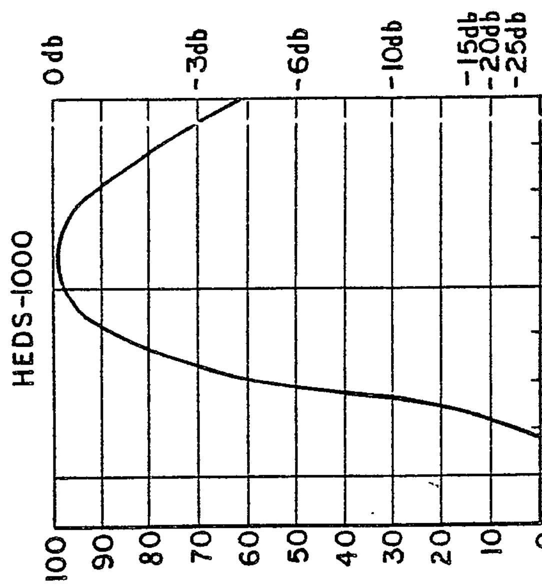


Fig_5

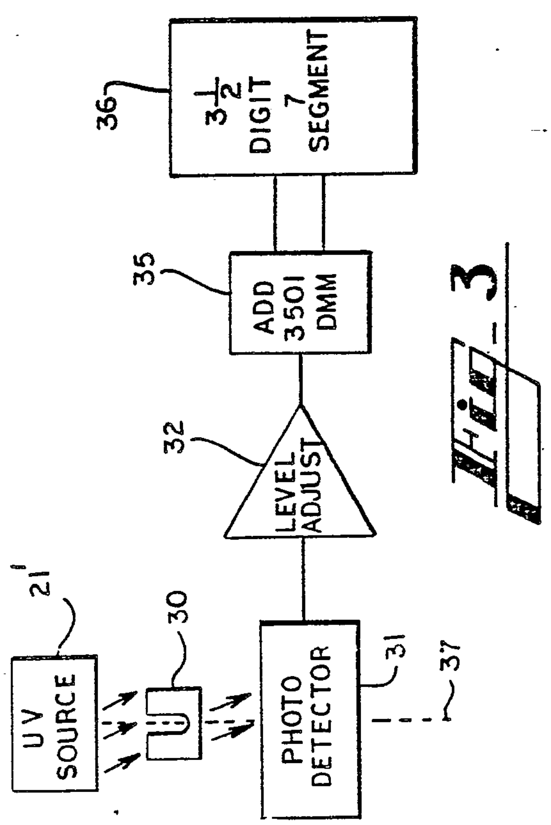


Fig_3

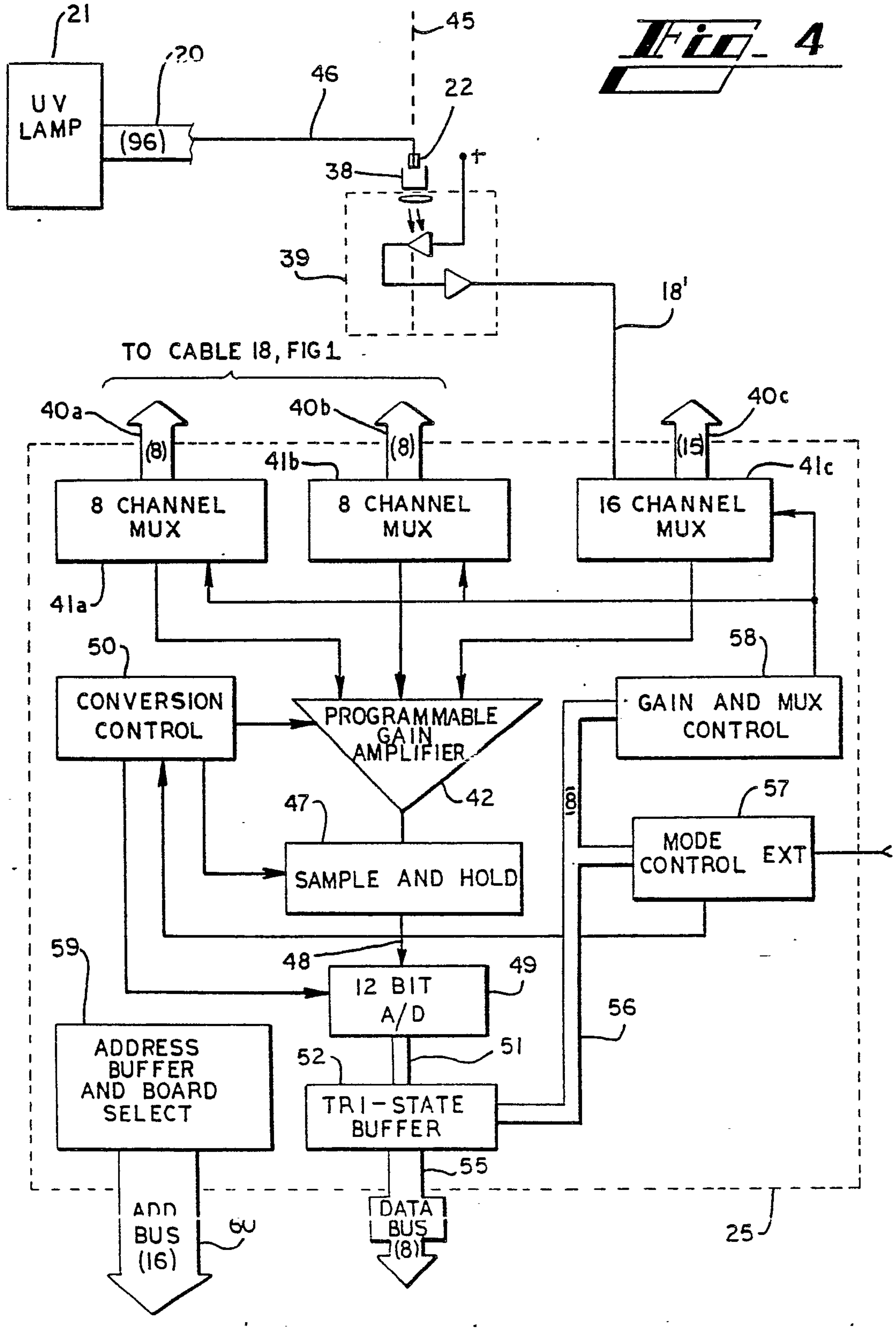
Fig_ 4
21
20
UV LAMP
(96)
46
45
22
38
39
18'
TO CABLE 18, FIG 1
40a
(8)
40b
(8)
40c
(15)
41b
41c
8 CHANNEL MUX
8 CHANNEL MUX
16 CHANNEL MUX
41a
50
58
CONVERSION CONTROL
PROGRAMMABLE GAIN AMPLIFIER
GAIN AND MUX CONTROL
42
47
57
(8)
MODE CONTROL
EXT
SAMPLE AND HOLD
59
48
49
12 BIT A/D
56
52
51
ADDRESS BUFFER AND BOARD SELECT
TRI-STATE BUFFER
55
ADD BUS (16)
DATA BUS (8)
25